# EUROPEAN PATENT SPECIFICATION

(11) **EP 2 018 122 B1**
(45) Date of publication and mention of the grant of the patent: **20.04.2011**
(21) Application number: 06755238.0
(22) Date of filing: 17.05.2006
(51) Int. Cl.: A61B 17/22, A61F 2/02, A61B 17/00, A61F 2/06, A61B 19/00

(54) **A CONTROLLABLE DEVICE AND KIT FOR TREATMENT OF DISORDERS IN THE HEART RHYTHM REGULATION SYSTEM**
KONTROLLIERBARE VORRICHTUNG UND KIT ZUR BEHANDLUNG VON STÖRUNGEN DES HERZRHYTHMUS-REGULIERSYSTEMS
DISPOSITIF CONTRÔLABLE ET KIT DESTINÉS À TRAITER DES TROUBLES DU SYSTÈME DE RÉGULATION DU RYTHME CARDIAQUE

(43) Date of publication of application: 28.01.2009
(73) Proprietor: Syntach AG, 8200 Schaffhausen (CH)
(72) Inventor: SOLEM, Jan Otto, CH-8234 Stetten (CH); NIELSEN, Stevan, 72108 Rottenburg Am Neckar (DE); JOERGENSEN, Ib, 72401 Haigerloch (DE); SEIBOLD, Gerd, 72119 Ammerbuch (DE); QUINT, Bodo, 72108 Rottenburg (DE)
(74) Representative: KIPA
(86) International application number: PCT/EP2006/062403
(87) International publication number: WO 2007/131553

(56) References cited:
- EP-A2- 1 290 987
- WO-A2-2004/078065
- US-A1- 2003 078 649

## Description

### Field of the Invention

The present invention relates to treatment of disorders in the heart rhythm regulation system and, specifically, to a tissue cutting device, and a kit of shape-changing devices.

### Background of the Invention

The circulation of blood in the body is controlled by the pumping action of the heart. The heart expands and contracts by the force of the heart muscle under impulses from the heart rhythm regulation system. The heart rhythm regulation system transfers an electrical signal for activating the heart muscle cells.

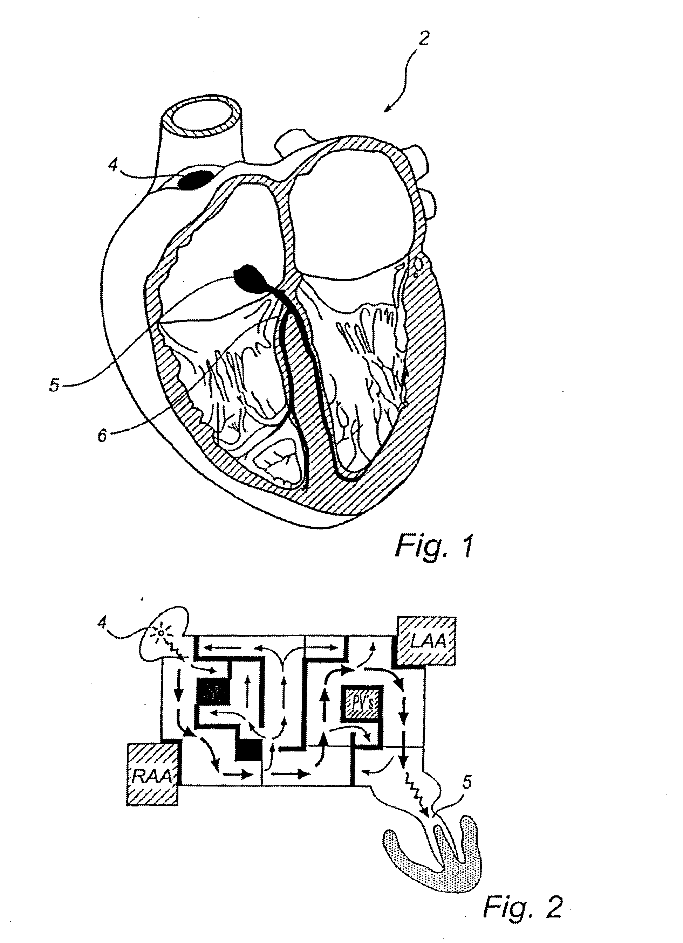

The normal conduction of electrical impulses through the heart starts in the sinoatrial node, travels across the right atrium, the atrioventricular node, the bundles of His and thereafter spread across the ventricular muscle mass. Eventually when the signal reaches the myocytes specialized in only contraction, the muscle cell will contract and create the pumping function of the heart (see Fig. 1).

The electrical impulses are transferred by specially adapted cells. Such a cell will create and discharge a potential over the cell membrane by pumping ions in and out of the cell. Adjacent cells are joined end-to-end by intercalated disks. These disks are cell membranes with a very low electrical impedance. An activation of a potential in a cell will propagate to adjacent cells thanks to the low impedance of the intercalated disks between the cells. While being at the embryonic stage, all heart muscle cells, the myocytes, have the ability to create and transfer electrical signals. During evolution the myocytes specialize and only those cells necessary for maintaining a stable heart-rate are keeping the ability to create and send electrical impulses. For a more thorough explanation of the propagation of electrical signals in the heart, see e.g. Sandöe, E. and Sigurd, B., Arrhythmia, Diagnosis and Management, A Clinical Electrocardiographic Guide, Fachmed AG, 1984.

The heart function will be impaired if there is a disturbance on the normal conduction of the electrical impulses. Atrial fibrillation (AF) is a condition of electrical disorder in the heart rhythm regulation system. In this condition, premature and fast signals irregularly initiating muscle contractions in the atria as well as in the ventricles will be started in ectopic sites, that is areas outside the sinoatrial node. These signals will be transmitted erratically all over the heart. When more than one such ectopic site starts to transmit, the situation becomes totally chaotic, in contrast to the perfect regularity in a healthy heart, where the rhythm is controlled from the sinoatrial node.

Atrial fibrillation is a very common disorder, thus 5% of all patients that undergo heart surgery suffer from AF. 0.4-2% of a population will suffer from AF, whereas 10 % of the population over the age of 65 suffers from AF. 160 000 new cases occur every year in the US and the number of cases at present in the US is estimated to be around 3 million persons. Thus, treatment of atrial fibrillation is an important topic.

Typical sites for ectopic premature signals in AF may be anywhere in the atria, in the pulmonary veins (PV), in the coronary sinus (CS), in the superior vena cava (SVC) or in the inferior vena cava (IVC). There are myocardial muscle sleeves present around the orifices and inside the SVC, IVC, CS and the PVs. Especially around the orifice of the left superior pulmonary vein (LSPV) such ectopic sites are frequent, as well as at the orifice of the right superior pulmonary vein (RSPV). In AF multiple small circles of a transmitted electrical signal started in an ectopic site may develop, creating re-entry of the signal in circles and the circle areas will sustain themselves for long time. There may be only one ectopic site sending out signals leading to atrial flutter, or there may be multiple sites of excitation resulting in atrial fibrillation. The conditions may be chronic or continuous since they never stop. In other cases there may be periods of normal regular sinus rhythm between arrhythmias. The condition will then be described as intermittent.

In the chronic or continuous cases, the atrial musculature undergoes an electrical remodelling so that the re-entrant circuits sustain themselves continuously. The patient will feel discomfort by the irregular heart rate, sometimes in form of cannon waves of blood being pushed backwards in the venous system, when the atria contract against a closed arterio-ventricle valve. The irregular action of the atria creates standstill of blood in certain areas of the heart, predominantly in the auricles of the left and right atrium. Here, blood clots may develop. Such blood clots may in the left side of the heart get loose and be taken by the blood stream to the brain, where it creates disastrous damage in form of cerebral stroke. AF is considered to be a major cause of stroke, which is one of the biggest medical problems today.

Today, there are a few methods of treating the problems of disorders to the heart rhythm regulation system. Numerous drugs have been developed to treat AF, but the use of drugs is not effective to a large part of the patients. Thus, there has also been developed a number of surgical therapies.

Surgical therapy was introduced by Drs. Cox, Boineau and others in the late 1980s. The principle for surgical treatment is to cut all the way through the atrial wall by means of knife and scissors and create a total separation of the tissue. Subsequently the tissues are sewn together again to heal by fibrous tissue, which does not have the ability to transmit myocardial electrical signals. A pattern of cutting was created to prohibit the propagation of impulses and thereby isolate the ectopic sites, and thus maintain the heart in sinus rhythm. The rationale for this treatment is understandable from the description above, explaining that there must be a physical contact from myocyte to myocyte for a transfer of information between them. By making a complete division of tissue, a replacement by non-conductive tissue will prohibit further ectopic sites to take over the stimulation. The ectopic sites will thus be isolated and the impulses started in the ectopic sites will therefore not propagate to other parts of the heart.

It is necessary to literally cut the atria and the SVC and the IVC in strips. When the strips are sewn together they will give the impression of a labyrinth guiding the impulse from the sinoatrial node to the atrioventricular node, and the operation was consequently given the name Maze. The cutting pattern is illustrated in Fig. 2 and was originally presented in JL Cox, TE Canavan, RB Schuessler, ME Cain, BD Lindsay, C Stone, PK Smith, PB Corr, and JP Boineau, The surgical treatment of atrial fibrillation. II. Intraoperative electrophysiologic mapping and description of the electrophysiologic basis of atrial flutter and atrial fibrillation, J Thorac Cardiovasc Surg, 1991 101: 406-426. The operation has a long-time success of curing patients from AF in 90 % of the patients. However, the Maze operation implicate that many suture lines have to be made and requires that the cuts are completely sealed, which is a demanding task for every surgeon that tries the method. The operation is time consuming, especially the time when the patients own circulation has to be stopped and replaced by extracorporeal circulation by means of a heart-lung machine. Thus mortality has been high and the really good results remained in the hands of a few very trained and gifted surgeons.

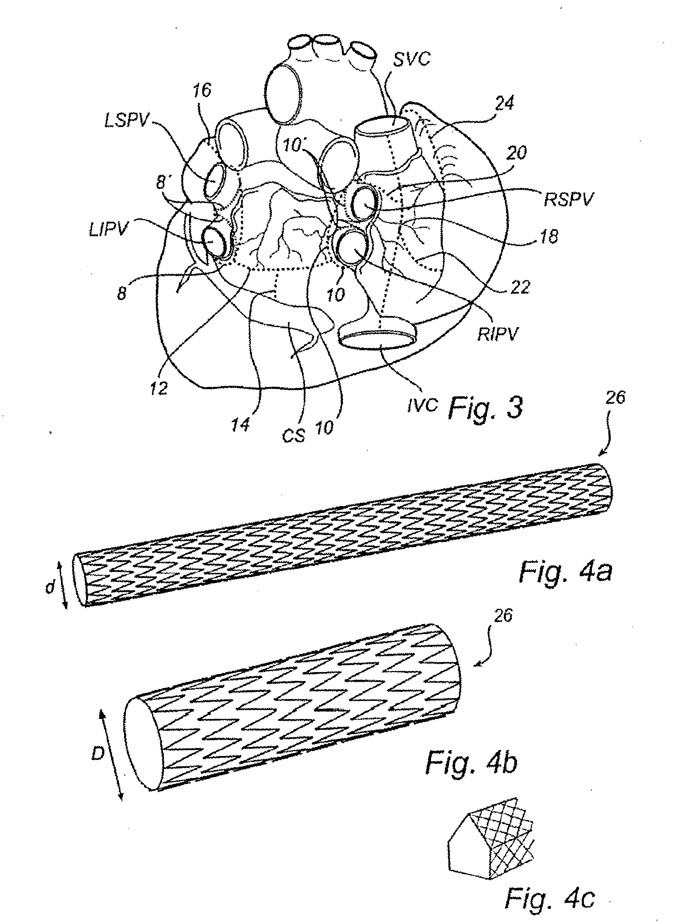

The original Maze operation has therefore been simplified by eliminating the number of incisions to a minimum, still resulting in a good result in most cases. The currently most commonly used pattern of incisions is called Maze III (see Fig. 3).

Other methods of isolating the ectopic sites have also been developed recently. In these methods, the actual cutting and sewing of tissue has been replaced by methods for killing myocyte cells. Thus, one may avoid separating the tissue, instead one destroy the tissue by means of heat or cooling in the Maze pattern to create a lesion through the heart wall. The damaged myocyte tissue can not transfer signals any more and therefore the same result may be achieved. Still the chest has to be opened, and the heart stopped and opened. Further, the energy source has to be carefully controlled to affect only tissue that is to be destroyed.

A large number of devices have now been developed using various energy sources for destroying the myocyte tissue. Such devices may use high radio frequency energy, as disclosed in e.g. US 5,938,660, or microwaves, ultrasound or laser energy. Recently, devices have been developed for catheter-based delivery of high radio frequency energy through the venous and or arterial systems. However, this has so far had limited success due to difficulties in navigation and application of energy and also late PV stenosis has been reported. Further, devices using cooling of tissue has used expanding argon gas or helium gas to create temperatures of -160°C. Using an instrument with a tip, tissue can be frozen and destroyed.

WO 03/003948 discloses an apparatus for treating, preventing, and terminating arrhythmias. The device, which is implanted and left at the target site, is provided with protrusions that pierce the tissue, via self-expansion or balloon expansion, to gain access to the cells of said target site. The protrusions are used to conduct drugs to the cells, which drugs may cause cell death to thereby induce cellular changes that may lead to treatment of arrhythmias. Nowhere in WO 03/003948 is a device described that by expansion fully penetrates the wall of the blood vessel to disrupt cardiac impulses, which device then is bio-absorbed and thereby eliminated from the target site. The device according to WO 03/003948 is not a cutting device.

WO 2004/078065 discloses a tissue cutting device according to the preamble of claim 1.

Also, prior art is silent about a cutting device, which is provided with means for regulating the cutting action, such that the cutting action is stopped or initiated when said means for regulating the cutting action is de-activated or activated, respectively.

Hence, there is a need for a more advantageous cutting device.

### Summary of the Invention

Accordingly, the present invention seeks to mitigate, alleviate or eliminate one or more of the above-identified deficiencies and to provide a new device, and kit of devices, suitable for a method for treatment of disorders to the heart rhythm regulation system of the kinds referred to, according to the appended independent claim 1.

For this purpose a tissue cutting device according to claim 1 is provided, wherein the device is structured and arranged to be inserted in a temporary delivery shape through the vascular system into a body vessel adjacent to the heart and/or into the heart and to be subsequently subjected to a change of shape, from said temporary delivery shape via an expanded delivered shape to a further expanded shape, in order to create cutting action configured for cutting said heart tissue and/or said body vessel, and wherein the device comprises at least one connection element, arranged such that the connection formed from said connection element between a first and second part of said cutting device is broken when said connection element is subjected to a specific external influence.

Advantageous features of the invention are defined in the dependent claims.

### Brief Description of the Drawings

The invention will now be described in further detail by way of example under reference to the accompanying drawings, on which:
Fig. 1 is a schematic view of the transmission of electrical signals in the heart;
Fig. 2 is a schematic view of a pattern of cutting tissue of the heart wall according to the Maze-procedure for treating disorders to the heart rhythm regulation system;
Fig. 3 is a schematic view of a simplified pattern according to the Maze III-procedure, wherein the heart is seen from behind;
Figs 4a-4c are perspective schematic views of a tissue cutting device according to an embodiment of the invention, wherein Fig. 4a shows the tissue cutting device in a first, temporary shape, Fig. 4b shows the tissue cutting device in a second, permanent shape, and Fig. 4c illustrates the tissue cutting device having sharp edges;
Fig. 5 (S005) is a schematic view of a configuration of connecting material,
Figs 6 a to d is a schematic view of an embodiment with a configuration that prevents expansion into an expanded state,
Fig. 7 is a schematic view of an embodiment in which the connection material is located in between two parts of the cutting device,
Fig. 8 (S010) is a schematic view of the connecting material connects separate spiral fragments in a cutting device of a spiral shape,
Fig. 9 a to d illustrates different embodiments of cutting devices with altering direction of cutting action,
Fig. 10 shows different embodiments of the tissue cutting device,
Figs 11-13 illustrate three different embodiments of accessing the vascular system;
Fig. 14 illustrates a guide wire being inserted into the coronary sinus;
Fig. 15 illustrates a guide wire being inserted into the coronary sinus and a guide catheter being inserted with its tip at the orifice of the coronary sinus;
Fig. 16 is a view similar to Fig. 15 showing a first tissue cutting device being inserted into the coronary sinus;
Figs 17 and 18 illustrate a guide wire having been inserted into the left atrium;
Figs 19-21 illustrate the carrying and deployment of a tissue cutting device by means of a delivery catheter;
Figs. 22-24 illustrate the deployment of a tissue cutting device in the left superior pulmonary vein;
Figs 25-28 illustrate the insertion of a tissue cutting device into the inferior and superior vena cava;
Fig 29 illustrate the deployment of a tissue cutting device according to Fig 10 in the left atrium;
Fig 30 illustrate the deployment of a tissue cutting device according to Fig 10 in the right atrium; and
Fig 31 illustrate a tissue lesion creating cutting device according to Fig 10a located in the left atrium.

### Detailed Description of Embodiments

Referring now to Figs 1-3, the problems of disorders to the heart rhythm regulation system and the leading current method of treating these problems will be described. In Fig. 1, a heart 2 is shown and the controlling of the heart rhythm is indicated. The heart rhythm is normally controlled from the sinoatrial node 4. The sinoatrial node 4 transmits electrical signals, which are propagated through the heart wall by means of special cells forming an electrical pathway. The electrical signals following the electrical pathway will coordinate the heart muscle cells for almost simultaneous and coordinated contraction of the cells in a heart atrium and heart ventricle. The normal conduction of electrical impulses through the heart starts in the sinoatrial node 4, travels across the right atrium, the atrioventricular node 5, the bundles of His 6 and thereafter spread across the ventricular muscle mass. In a disordered situation, electrical signals are started in heart cells outside the sinoatrial node 4, in so-called ectopic sites. These electrical signals will disturb the coordination of the heart muscle cells. If several ectopic sites are present, the signal transmission becomes chaotic. This will be the cause of arrhythmic diseases, such as atrial fibrillation and atrial flutter.

An existing method for treating these diseases is based on isolating the ectopic sites in order to prevent the electrical signals started in these ectopic sites to propagate in the heart wall. Thus, the heart wall is cut completely through for interrupting the coupling between cells that transmit erratic electrical signals. The thus created lesion will be healed with fibrous tissue, which is unable to transmit electrical signals. Thus, the path of the electrical signals is blocked by this lesion. However, since the location of the ectopic sites may not always be known and may be difficult to determine or since there might be multiple ectopic sites, a special cutting pattern has been developed, which will effectively isolate ectopic sites. Thus, the same pattern may always be used regardless of the specific locations of the ectopic sites in each individual case. The procedure is called the "Maze"-procedure in view of the complicated cutting pattern. In Fig. 2, the Maze-pattern is illustrated.

However, as is evident from Fig. 2, the cutting pattern is extensive and complex and requires a difficult surgery. Thus, the Maze-pattern has been evolved in order to minimize the required cuttings and simplify the pattern as much as possible. Currently, a Maze III-pattern is used, as shown in Fig. 3. This pattern is not as complicated, but would still effectively isolate the ectopic sites in most cases. The Maze III-pattern comprises a cut 8 around the left superior pulmonary vein (LSPV) and the left inferior pulmonary vein (LIPV) and a corresponding cut 10 around the right superior pulmonary vein (RSPV) and the right inferior pulmonary vein (RSPV); a cut 12 connecting the two cuts 8 and 10 around the pulmonary veins (PV); a cut 14 from this connecting cut to the coronary sinus (CS); a cut 16 from the left PVs to the left atrial appendage; a cut 18 from the inferior vena cava (IVC) to the superior vena cava (SVC); a cut 20 connecting the cut 10 around the right PVs and the cut 18 between the IVC and the SVC; a cut 22 from the cut 18 between the IVC and the SVC along the right lateral atrium wall; and a cut 24 isolating the right atrial appendage. Thus, a pattern, which is less complex and which effectively isolates the ectopic sites, has been established. In some cases, all cuts may not be needed. For example, the occurrence of ectopic sites often starts around the orifices of the PVs and, therefore, it may be sufficient to make the cuts 8, 10 around the PVs. Further, as indicated with the lines 8' and 10', the cuts around the PVs may be done along each PV orifice instead of in pairs.

According to the invention, there is provided a possibility of cutting through the heart wall in a new manner. Thus, a similar pattern to the Maze III-pattern should also be achieved according to this new manner. However, as mentioned above, it may not in all cases be required that all cuts of the Maze III-pattern are made. Furthermore, some cuts may be preferably interrupted after some time of cutting, to not cut tissue in the vicinity of the tissue to be cut, or if only a part of the tissue is to be cut, for example if the ectopic site to be isolated is located close to surface first subjected to cutting. It may also be possible to activate a cutting action after some time of subjection to a stimuli by providing said cutting device which

An already filed published international application, of same applicant as the present application, with publication number WO 2006/122573, a heart wall tissue lesion creating cutting device is described and the new manner of performing the cuts through the heart wall is explained, which international application hereby is integrated herein in its entirety. This heart wall tissue lesion creating cutting device 26 (hereinafter called cutting device) is shown in Fig. 4a in a first state, in which the cutting device 26 is tubular and has a first diameter d. The cutting device 26 is shown in Fig. 4b in a second state, in which the cutting device 26 is tubular and has a second diameter D, which is larger than the first diameter d. The cutting device 26 is formed of a shape memory material, which has the ability of memorizing a permanent shape that may significantly differ from a temporary shape. The shape memory material will transfer from its temporary to its memorized, permanent shape as a response to a suitable stimulus. The stimulus may be exposure to a raised temperature, such as a temperature above e.g. 30°C that may be caused by the body temperature. The stimulus may suitably be combined with the release of a restraining means, which may keep the shape memory material from assuming its permanent shape.

Thus, the cutting device 26 may be inserted in this temporary shape to the heart of a patient through the vascular system. The temporary shape of the cutting device 26 is also flexible, whereby guiding the cutting device 26 through the vascular system is facilitated. This insertion of the cutting device 26 may be performed with well-known percutaneous catheter techniques. This is an unaggressive procedure and may be performed on a beating heart. Thus, the cutting device 26 may readily be positioned at a desired position within the vascular system adjacent heart wall tissue to be treated. The cutting device 26 may then be allowed to transfer to its memorized, permanent shape when inserted to the desired position in a blood vessel.

The memorized, permanent shape of the cutting device 26 will not fit into the blood vessel 28, whereby the cutting device 26 will force itself through surrounding tissue for obtaining the permanent shape. In this way, the cutting device 26 will first penetrate the vessel wall and thereafter tissue surrounding the blood vessel 28. Tissue cells that are penetrated will be killed, which will start a healing reaction in the body. Where the cutting device 26 is placed in a desired position to change shape through heart wall tissue, cells that are able to transmit electrical signals may thus be killed. The healing process will not restore the ability to transmit electrical signals and, therefore, the cutting device 26 will reduce the ability of transmitting electrical signals through the heart wall.

An example of a shape memory material is Nitinol, which is an alloy composed of nickel (54-60%) and titanium. Small traces of chrome, cobalt, magnesium and iron may also be present. This alloy uses a martensitic phase transition for recovering the permanent shape. Shape memory materials may also be formed of shape memory polymers, wherein the shape-memory effect is based on a glass transition or a melting point. Such shape memory polymers may be produced by forming polymers of materials, or combinations of materials, having suitable properties. For example, a shape memory polymer may be created of oligo(e-caprolactone) dimethacrylate combined with n-butyl acrylate. Also, biodegradable, bioresorbable, or bioabsorbable materials may be used for forming these shape memory polymers. In this way, the cutting device 26 may be designed such that it will be degraded or absorbed by the body after it has performed its change of shape. For example, a polylactic acid polymer and/or a polyglycolic acid polymer, poly (e-caprolactone) or polydioxanone may be used for forming a shape memory polymer that is biodegradable. A special feature of the resorbable shape memory polymers is that these will disappear from the tissue after having had its function, limiting potential negative effects of otherwise remaining polymer or Nitinol materials, such as perforations and damage to other adjacent tissues, like lungs, oesophagus and great vessels like the aorta.

According to the present invention means for deactivating and/or activating at least a part of said cutting device is provided, which may interrupt, after some time of cutting, the cutting action of said cutting device, or initiate cutting action after some time of presence at preferred site. In this respect the cutting device is provided with a connecting material, which connecting material may be broken, dissolved, absorbed, or in any other way effected in such way the connection between the two parts bound together with said connecting material is broken. This may for example be accomplished with a bio-absorbable material, which, when affected by a biologic environment, is absorbed, to thereby break the connection between the two parts bound together with said connecting material is broken. This may also be accomplished with a stress breaking material. If the connecting material is a stress breaking material, the connection may be broken after some time in a stress generating environment, such as in the heart, i.e. atrium, ventricle etc., or in the vascular system. When the connection is broken between two parts of the cutting device, the cutting action may either be interrupted or initiated.

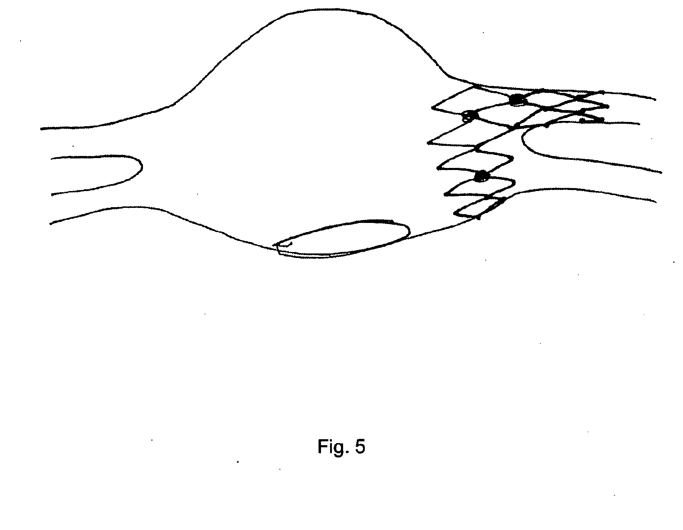

In one embodiment, according to Fig. 5 (S005), the connecting material connects two wires of the cutting device. When the connecting material is broken, such as through absorption if the connecting material is a bio-absorbable material, the two wires are disconnected. Thus, the transformation from a first state, for example in which the cutting device 26 is tubular and has a first diameter d to a second state, for example in which the cutting device 26 is tubular and has a second diameter D, which is larger than the first diameter d, is interrupted. Thereby, also the cutting action is interrupted.

In another embodiment the connecting material only connect parts in one part of the cutting device 26. Thereby, the cutting action may proceed in one part of the cutting device 26, while the cutting action is interrupted in another part of the cutting device 26. This may for example be advantageous if a part of the tissue surrounding the cutting device is intended to be cut only partly. An example could be if sensitive tissue is located adjacent to the tissue to be cut, whereby one wishes to secure that no cutting in said sensitive tissue is performed.

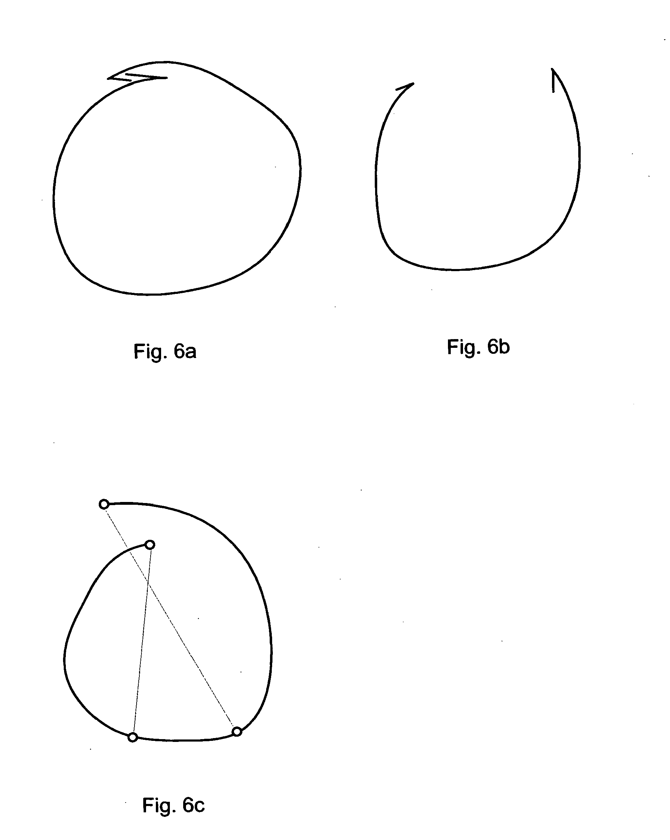

In still another embodiment the connecting material prevents the cutting device 26 from expanding to the memorized permanent shape. Thus, the transformation from a first state to a second state may be delayed. When the connection formed by the connecting material is broken, such as from absorption, stress etc., the cutting device 26 will be free to expand to said second state. In this embodiment the connecting material has to be located such that it connects two parts of the cutting device in such way that the connection prevents expansion into said second state. This may for example be accomplished with the arrangement according to Figs 6 a to d.

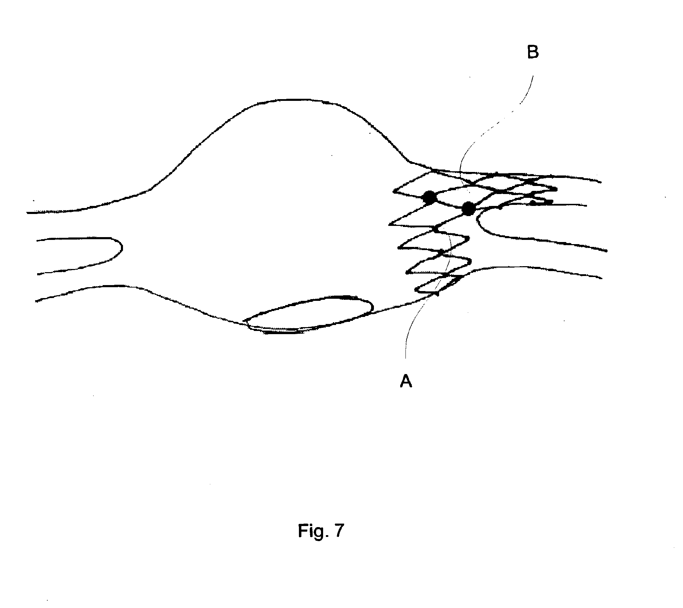

In one embodiment the connecting material is configured in accordance with Fig. 7. In this configuration the connection material is located in connective points of wires between a part A, for example cutting device to be located inside the atrium, and a part B, for example a "sock" to be located in an adjacent vascular vessel, whereby the sock constitutes a fixation means. Thereby, the cutting device may be fixated, by the aid of part B, such that part A performs cutting action at a preferred site in the atrium. When part A has expanded enough to start cutting action, one may wish to disrupt the cutting action of part B. The connecting material may therefore be such that the connection is broken after a suitable period of time, i.e. when part A has started to perform cutting action. Thus, the expansion into the memory shape of part A may continue, while part B maybe already has reached its memory shape. It is also possible to arrange the connecting material such that not only part A and part B are separated, but also part B is completely broken. The expansion of part B into its memory shape is therefore disrupted, and no further cutting action will be performed.

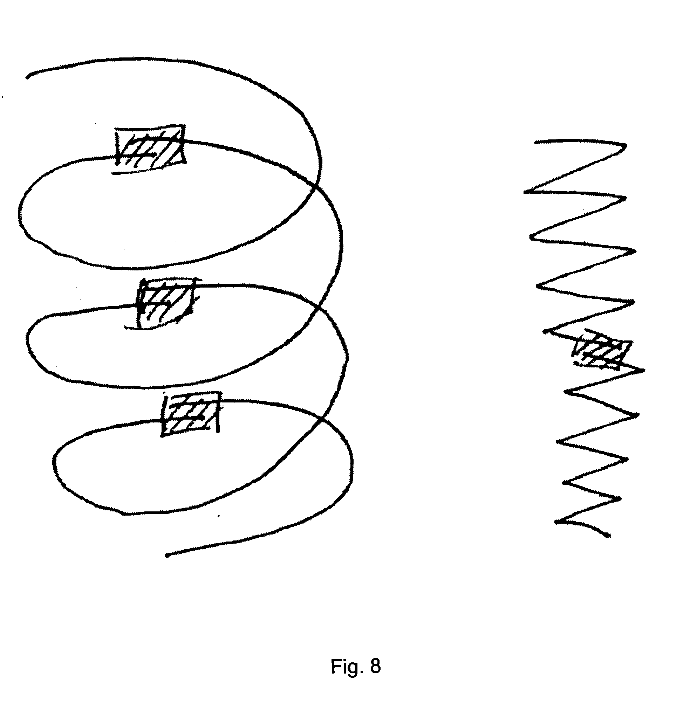

In another embodiment, wherein the cutting device is of a spiral shape, according to Fig. 8 (S010), the connecting material connects separate spiral fragments C and D. Thus, when the connecting material is absorbed, or broken by stress, the spiral fragments C and D are separated. The separation of spiral fragments, such as fragments C and D, will result in the disintegration of the cutting device, whereby the expansion into the memory shape, i.e. the cutting action, is disrupted.

The connecting material may also be made of any other material, that break under the influence of blood or other aqueous solutions, such as sugar based glues. In this example the breakage of the connection constituted by said connecting material is obtained by dissolution effect.

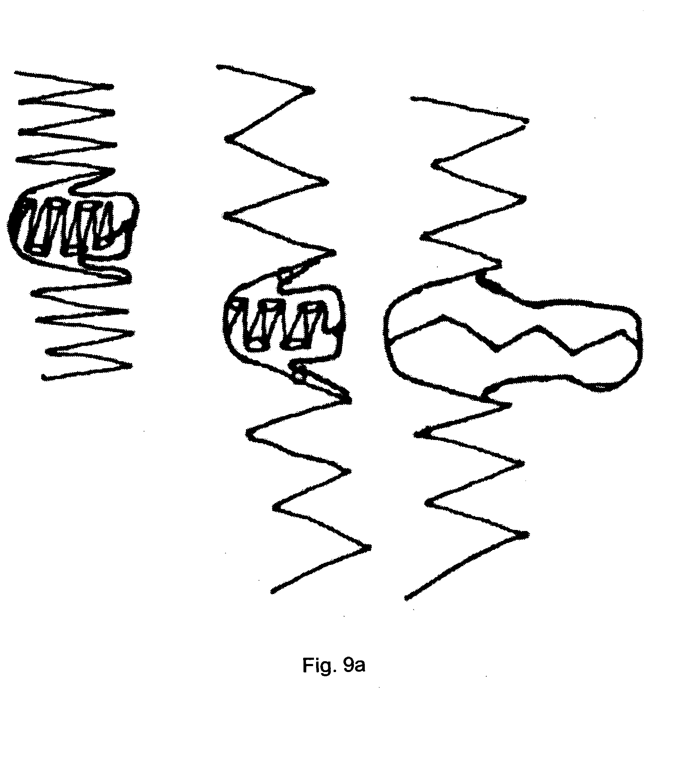

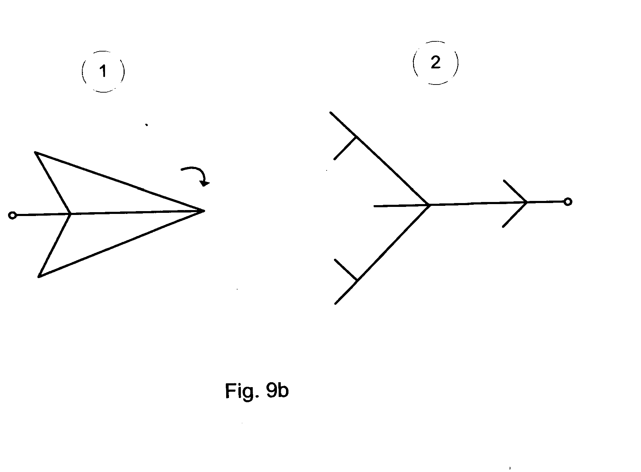

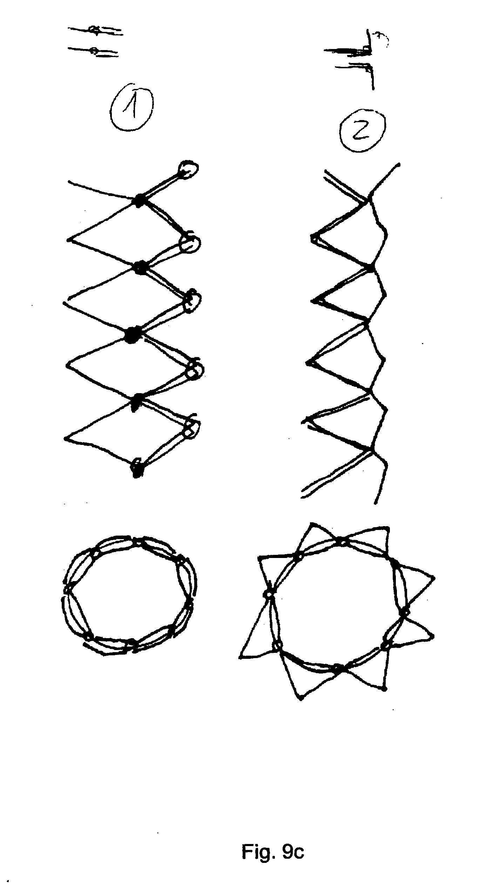

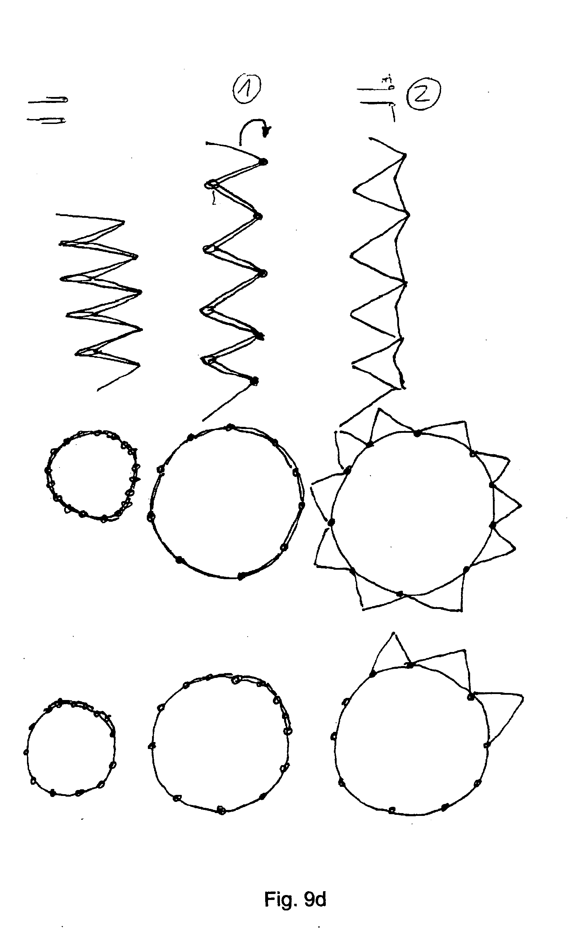

A similar effect, as with the connecting material, may be accomplished if wires of the cutting device are made thinner or welded together with a weld with low stress resistance. Thereby, the thinner parts of the cutting device may be broken by for example stress, to thereby interrupt the cutting action of the cutting device. It is possible to arrange the connecting material in such way that the direction of the cutting action of the cutting device is altered in time. A cutting device may for example have one direction of a cutting action until the breakage of a first connecting material initiates a change in direction. One may for example visualize a tubular cutting device with a first set of connection points, aligned in a manner that will break the tube into a sheet after the breakage of said connection points. Thus, Said sheet will have a different direction of the cutting action than the tubular cutting device. Thereafter, a second set of connection points, with a somewhat later breakage time, may break. This may lead to a second change of direction in respect of the cutting action. If we visualize the sheet obtained by the breakage of the first set of connection points, the second set of connection points may be localized in the centrum of said sheet. The breakage of this second set of breakage points will then initiate a second change of direction in respect of the cutting action, i.e. when the cutting action strives towards a globular form. Different embodiments of cutting devices with altering direction of cutting action are shown in Fig. 9 a to d.

In still another embodiment of a cutting device a spring or a material with a temporary delivery shape and a memory shape, is embedded in a bioresorbable and/or bioadsorbable material, which bioresorbable and/or bioadsorbable material prevents the spring or a material with a temporary delivery shape and a memory shape from expanding.

The cutting device 26, comprising said connecting material, may alternatively be formed to exhibit an elasticity such that it has a strive towards its permanent shape. This may be accomplished by forming the cutting device 26 to a spiral-shape in e.g. stainless steel or a magnesium alloy, which is biodegradable.

The cutting device 26 may be constructed of a net; i.e. its shape may comprise meshes or loops, which connection points between the wires in said net are connected by said connecting material or being thinner than said wires. This implies that a solid surface need not penetrate tissue, whereby the penetration through tissue and the forming of different shapes of the cutting device 26 will be facilitated.

The cutting device may also comprise one or more cutting arms (not shown), which, in the temporary shape of the cutting device, extend along a tubular part 32 or in an axial direction of the tubular part 32. Further, the cutting device may be arranged to change shape such that the one or more cutting arms extend in a radial direction from the tubular part. Thus, during the change of shape, the one or more cutting arms will penetrate through the tissue intended to be cut. The connection points within said arm(s) and/or between said arm(s) and said cutting device may be constituted by connecting material. Thereby, the arms may be broken off, or disintegrated when said connecting material is absorbed or broken by stress.

It is also possible to interconnect several cutting devices with wires, comprising connection points of connecting material, welds of low stress resistance, or a thinner part. These connection points may thus be broken, in the same manner as described above, to separate the several cutting devices. The advantage may be that the individual cutting devices fixates the position of eachother when the separate cutting devices not yet has initiated cutting action. This may for example be the case if one cutting device is intended to be located in one part of an atrium while another cutting device is intended to be located in another part of said atrium. By the aid of the wires, interconnecting said several cutting devices, the cutting devices may be fixated in the desired position until cutting action has been initiated. Thereafter the wires, connecting the cutting devices have no further use, whereby it may be preferable to break to connection between said several cutting devices. Otherwise, the wire, interconnecting said cutting devices, may influence the further cutting action of said cutting devices.

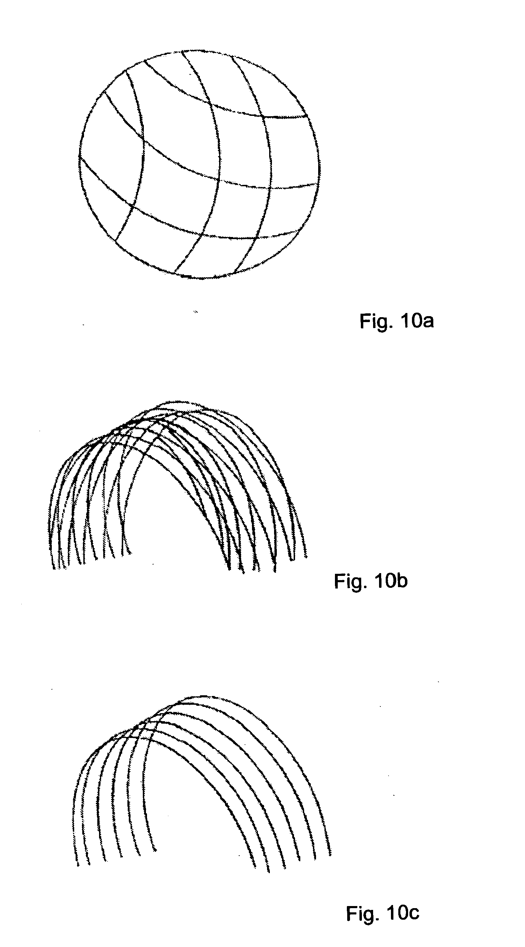

In the embodiment according to Fig. 10a the cutting device may be in form of a globulus. This globulus is placed inside the heart, such as in the left or right atrium, in a temporary shape. The cutting device is then stimulated, by for example temperature, according to above, to expand towards its memorized, permanent shape. This expansion results in that the heart tissue is cut by the cutting device. Tissue cells that are penetrated by the cutting device will be killed, which will start a healing reaction in the body. Where the cutting device is placed in a desired position to change shape through heart wall tissue, cells that are able to transmit electrical signals may thus be killed. The healing process will not restore the ability to transmit electrical signals and, therefore, the cutting device will reduce the ability of transmitting electrical signals through the heart wall. The globulus is provided with connecting material, which connecting material interconnects the wires constituting said cutting device. After some time the connecting material is absorbed or broken, by for example stress, whereby the expansion into its memorized, permanent shape is disrupted.

In another embodiment other parts than the "natural" interconnection points of the wires, which "natural" interconnection points may be arranged such that the globulus may expand into its memorized, permanent shape, are connected by the connection material. In this way the expansion of the cutting device may be prevented until the connection material is absorbed or broken, by for example stress.

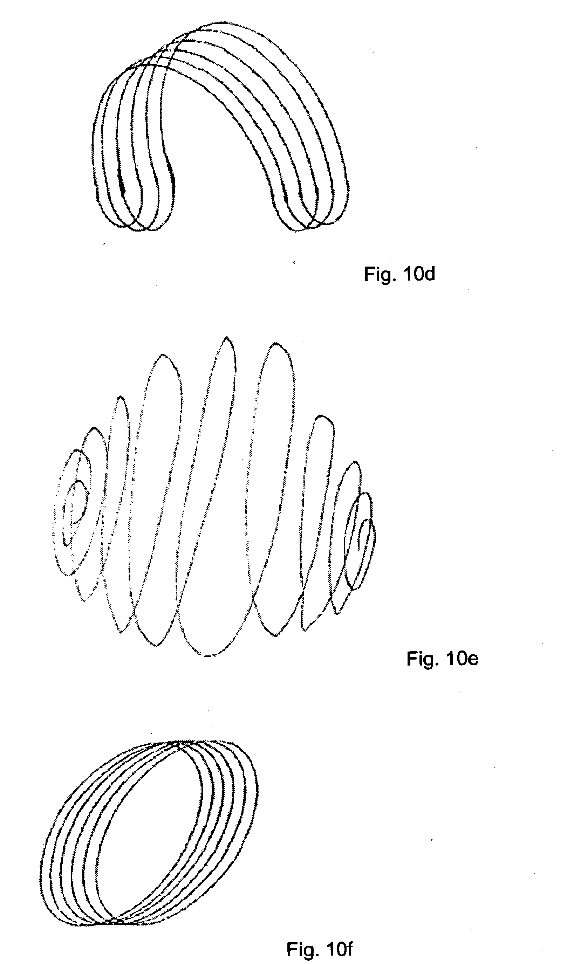

The cutting devices according to Fig. 10 may also be combined with the tubular parts of all other embodiments of the present invention, i.e. the cutting devices according to Fig 10 may be connected with different kinds of tubular parts. These tubular parts may then for example be delivered in a body vessel adjacent the heart while the cutting device according to Fig. 9 is delivered inside the heart.

Now, a system for delivery of a cutting device into a desired position in a blood vessel adjacent the heart will be described. Each cutting device may be inserted into its desired position using such a delivery system. The delivery system allows a precise placement of each cutting device into the heart and the big vessels of the body. The delivery system has a restraining device, which keeps the cutting device in its temporary shape. This allows insertion into the blood vessel through catheters having a small bore, making minimal trauma to the patient. The restraining device may be a restraining tube, into which the cutting device is forced in its temporary shape. By cooling the cutting device, in case of a cutting device made of Nitinol, it may be easier to force the cutting device into the restraining tube. Once inserted into the desired position, the cutting device may be pushed out of the restraining tube by means of a piston or the cutting device may be released by retracting the restraining tube from its position over the cutting device. In case of a cutting device made of Nitinol, the cutting device may also be restrained by cooling to prevent it from obtaining a transition temperature trigging the change of shape. Thus, the cutting device may be restrained by cooling during insertion into the desired position and released by suspension of the cooling when inserted at the desired position. In WO 03/022179, such a delivery system is described in more detail.

Now, a method for treating a patient having a disorder to the heart rhythm regulation system will be described. The patient is prepared for operation and operation is performed in an environment allowing visualization of the heart and the attached big vessels using fluoroscopy and ultrasound according to conventional techniques.

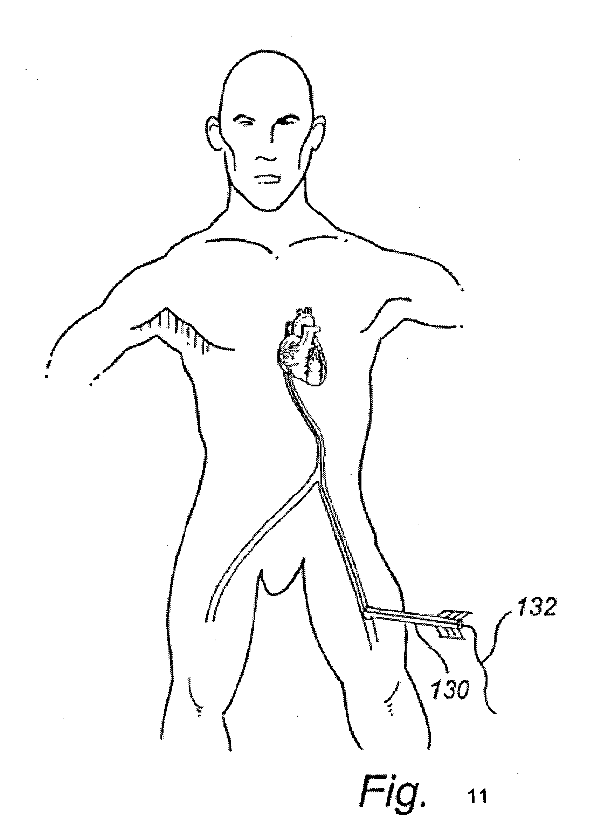

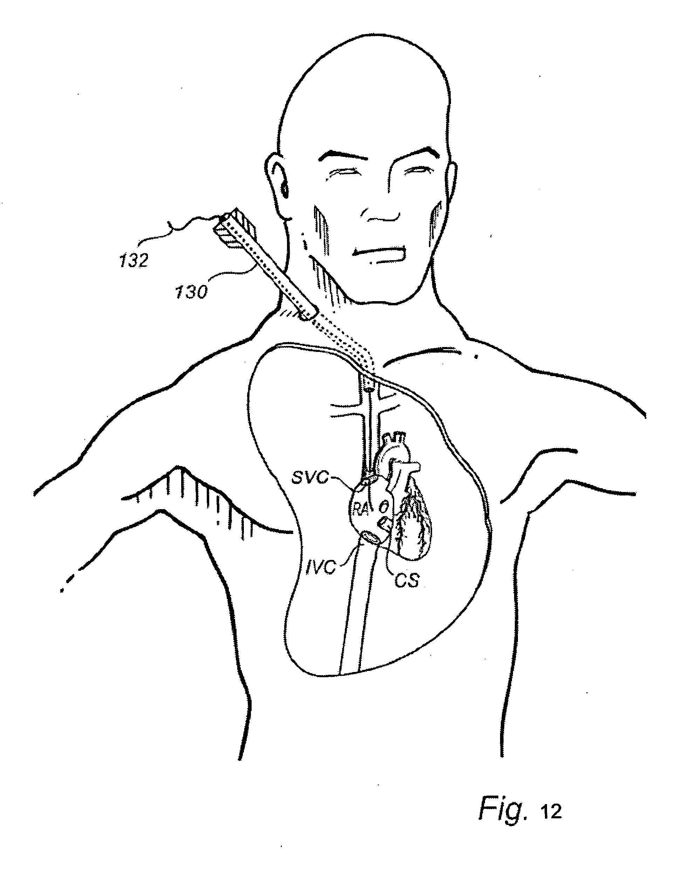

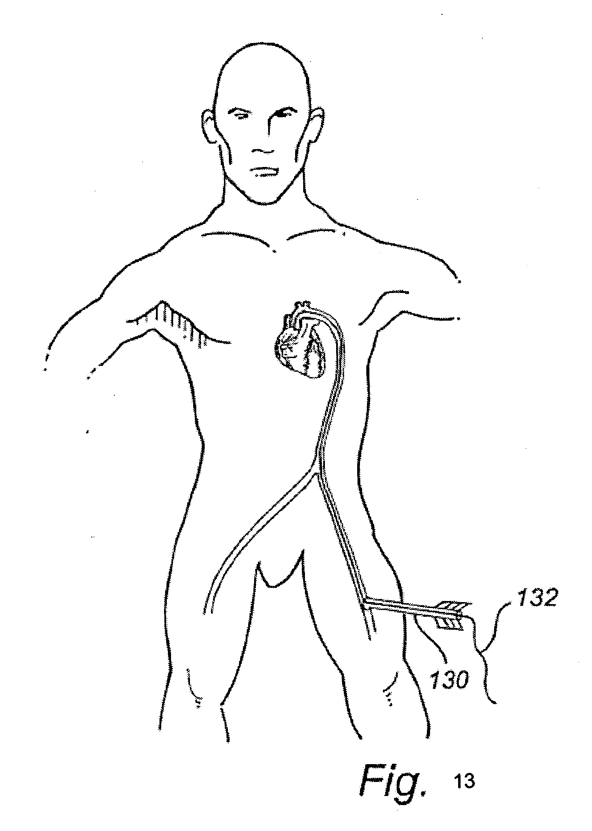

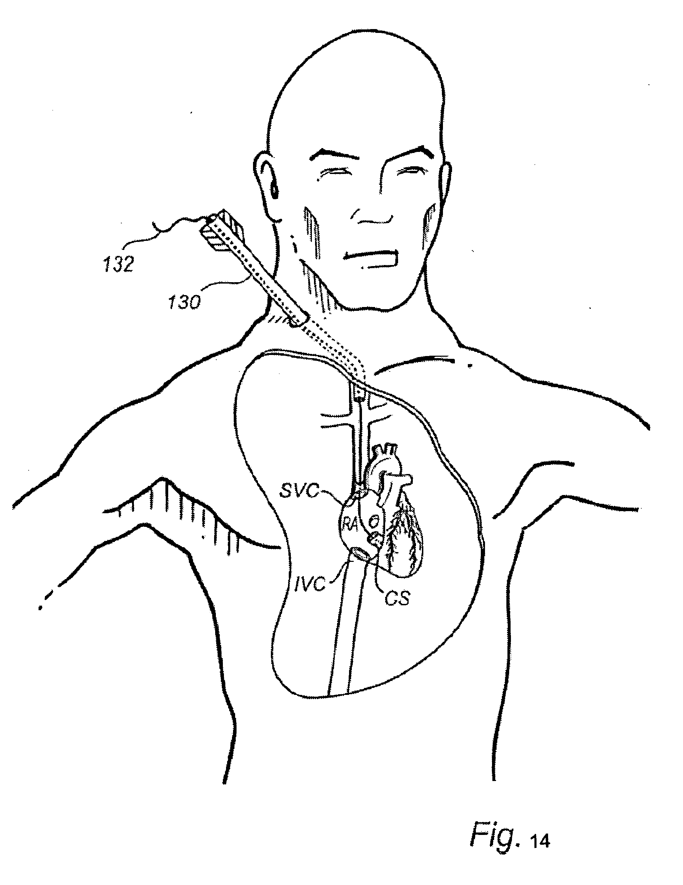

The operation is started by making a puncture of a vein providing an access point to the vascular system of the patient according to conventional techniques. Usually, the femoral vein in the groin, as illustrated in Fig. 11, the subclavian vein on the chest, or the internal or external jugular vein on the neck, as illustrated in Fig. 12, is used. However, other smaller veins may be used instead. Also, in difficult cases when the pulmonary veins cannot be accessed from the vein, arterial access through the femoral artery in the groin may be used, as illustrated in Fig. 13. This method will, however, not be further discussed here. A delivery system is used for inserting the above described cutting devices into blood vessels adjacent the heart. First, an introducer sheath 130 of the delivery system is inserted at the puncture providing an access route into the vascular system. Then, a diagnostic catheter of the delivery system is inserted through the introducer sheath 130 into the vascular system. The diagnostic catheter is manoeuvred through the vascular system into the CS. Next, a guide wire 132 of the delivery system is inserted through a channel of the diagnostic catheter into the CS and all the way to the vein parallel to the left anterior descending artery of the heart, close to the apex of the heart. The guide wire 132 is inserted as far as possible into the vascular system to be firmly positioned. Thereafter, the diagnostic catheter is withdrawn from the patient. The guide wire 132 will then extend from outside the patient into the patient via the access point and inside the patient to the CS, as illustrated in Fig. 14.

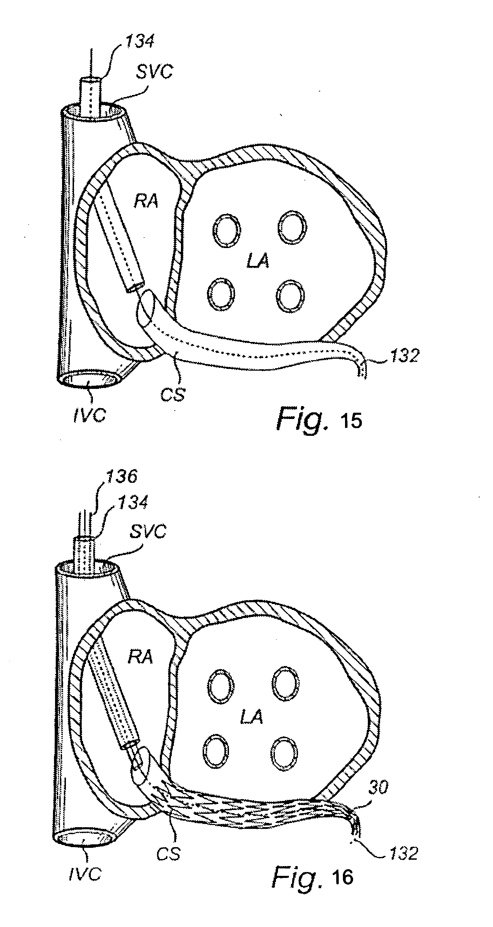

A guide catheter 134 of the delivery system is now inserted over the guide wire 132 so that the guide catheter 134 is positioned with its tip at the orifice of the CS, as illustrated in Fig. 15. Now, there is a guide wire 132 extending from the outside of the patient and the guide catheter 134, through the guide catheter 134, through the CS, the great cardiac vein and the anterior vein parallel to the LAD all the way to the apex of the heart.

Referring to Fig. 16, a delivery catheter 136 of the delivery system for carrying the first cutting device 30 into the desired position has a guide wire channel throughout its length. The end of the guide wire 132 outside the patient is then inserted into the guide wire channel of the delivery catheter 136, whereby the delivery catheter 136 may be inserted over the guide wire 132 and inside the guide catheter 134 into the CS. The delivery catheter 136 has an inner part providing the guide wire channel and carrying the cutting device at a distal portion. The delivery catheter 136 may further comprise an outer, restraining part, which covers the cutting device and keeps it in a contracted, temporary state. The restraining part may be axially displaceable in relation to the inner part. Thus, the restraining part may be retracted for releasing the cutting device. In this way, the first cutting device 30 is inserted into the CS and may be located in its desired position. A correct position is when the distal end 34 of the first cutting device 30 is positioned within the CS beyond the LIPV next to the CS and the proximal end 36 of the first cutting device 30 is closer to the orifice of the CS than the RIPV. Preferably, the first cutting device 30 extends all the way to the orifice of the CS. Without moving the first cutting device 30 away from its correct position, the first cutting device 30 is released from the delivery catheter. The first cutting device 30 will then immediately expand radially until contact is established with the CS wall, as illustrated in Fig. 16. Thereafter, the delivery catheter 136 is withdrawn from the patient.

However, the first cutting device 30 is arranged to change shape to assume a shape having much larger diameter than the natural diameter of the CS. Thus, the first cutting device 30 will expand to its designed, permanent shape and the CS wall will not be able to prevent the first cutting device 30 from obtaining its permanent shape. In order to obtain its permanent shape, the first cutting device 30 will therefore penetrate tissue in the path of the change of shape. In this way, the first cutting device 30 will expand to penetrate the heart tissue outside the CS, for instance the left atrium wall. The penetrated tissue will be killed and replaced by fibrous tissue, which is not able to transmit electrical signals. Thus, a block against propagation of undesired electrical signals may be created in this manner.

As an option, the first cutting device 30 may be inserted into the CS in a first separate session of the treatment of a patient. Thus, this first cutting device 30 may be allowed to be well-anchored in the tissue around the CS, before other cutting devices are inserted. This is suitable since some of the other cutting devices are adapted to contact the first cutting device 30 inserted into the CS in order to stabilize and fix their positions. The first cutting device 30 will be well-anchored within a few weeks, typically within three weeks. In this time the first cutting device 30 has penetrated the tissue around the CS and is firmly embedded by the tissue fixing its position. Then, the patient will come back for a second session of the treatment. Thus, a puncture is again made into a vein for allowing access again to the vascular system. However, all the cutting devices may alternatively be inserted during one session.

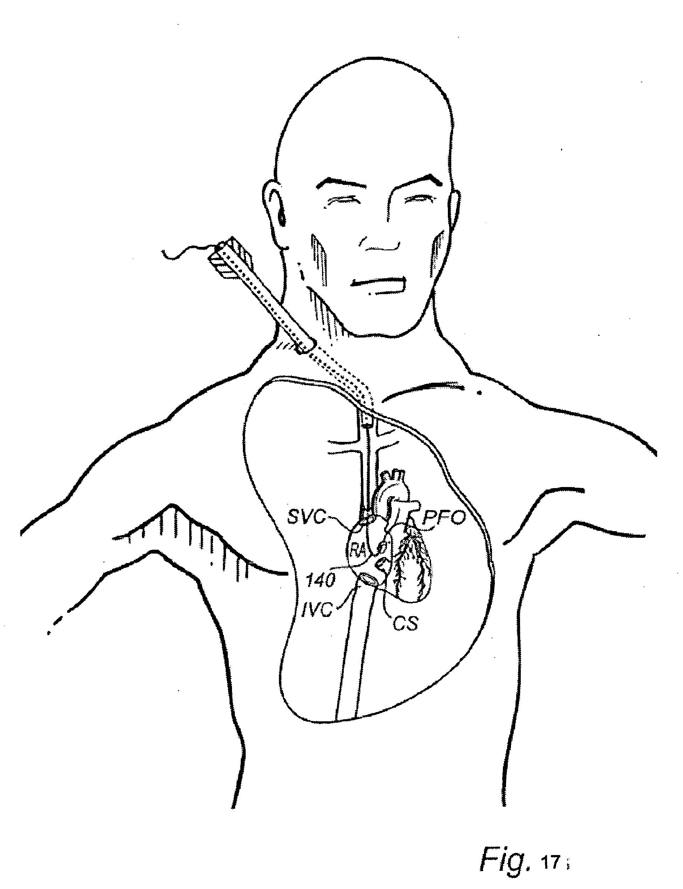

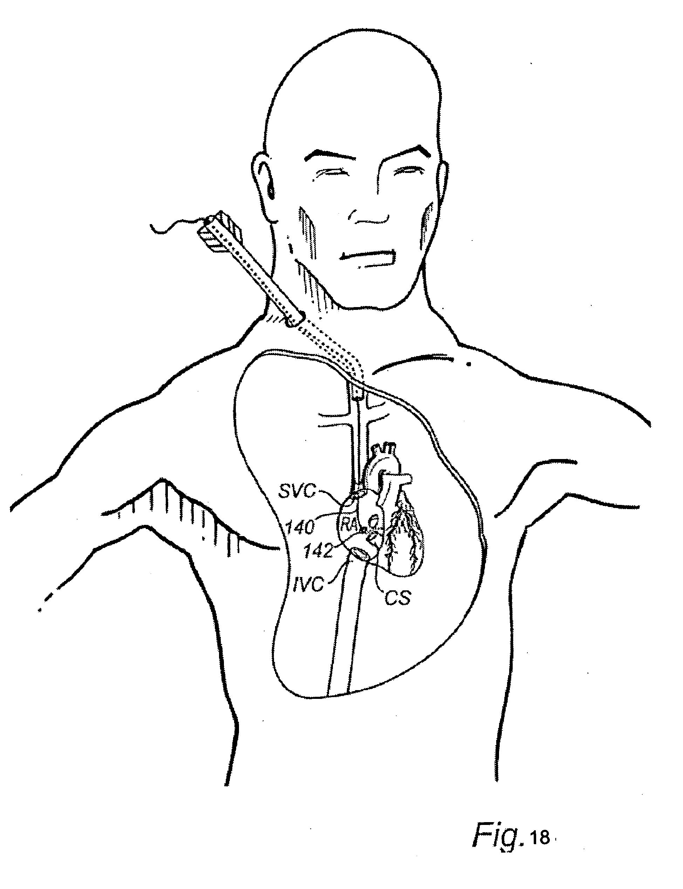

Now, a guide wire 140 is advanced inside a diagnostic catheter into the left atrium (LA), as illustrated in Figs 17 and 18. In order to access the LA, the atrial septum between the LA and the right atrium (RA) must be penetrated. If the patient has a patent foramen ovale (PFO, Fig 17), which is an opening between the LA and the RA that is normally only present during the fetal period in humans, this may be used and enlarged, for instance by means of a balloon catheter (not shown). If no PFO is present (Fig 18), a small opening 142 must first be created by means of a long flexible needle passed through a diagnostic catheter inside the access vein. Again, the opening 142 in the atrial septum may be enlarged by means of a balloon. Once the needle is inside the LA, the catheter is passed over the needle into the LA and the needle is retracted. A guide wire 140 may now be advanced through the catheter into the LA and further into the LIPV.

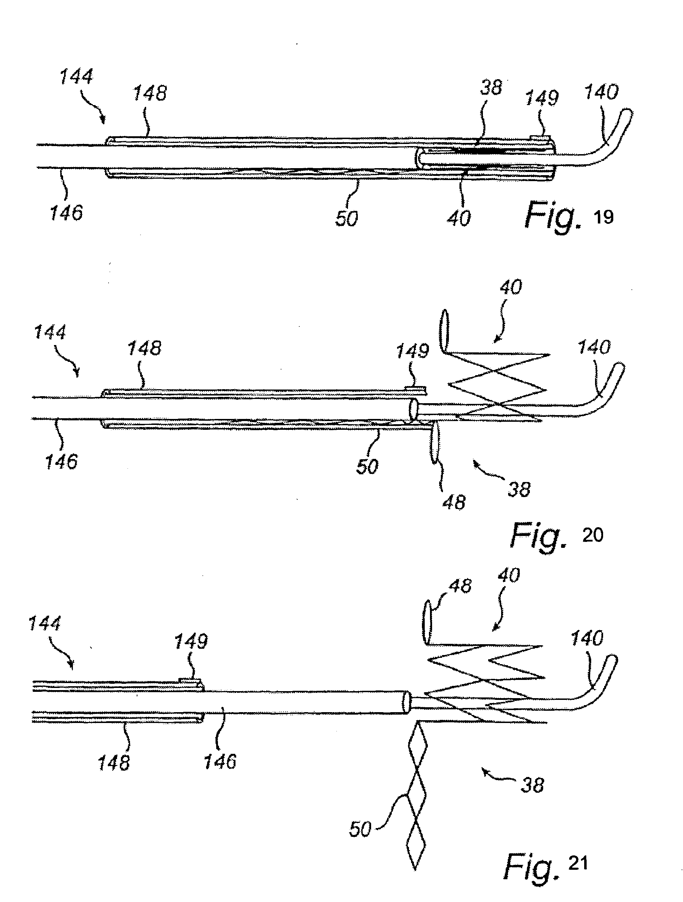

Referring now to Figs 19-21, the release of a cutting device will be generally described. Thus, having now placed the guide wire 140, the second cutting device 38 may be inserted to its desired position using a guide catheter extending to the LIPV orifice and a delivery catheter 144, as illustrated in Fig. 19, in a similar manner as for the insertion of the first cutting device 30. The delivery catheter 144 has an inner part 146 providing the guide wire channel. The tubular part 40 of the second cutting device 38 is arranged in front of the inner part 146 such that the inner part 146 of the delivery catheter 144 pushes the tubular part 40 in front of it. The delivery catheter 144 may further comprise an outer, restraining part 148, which covers the cutting device and keeps it in a contracted, temporary state. The restraining part 148 may be axially displaceable in relation to the inner part 146. Thus, the restraining part 148 may be retracted for releasing the cutting device 38. The delivery catheter 144 has a marker on the catheter outside the patient, as well as a x-ray marker 149 visible on the fluoroscopy, indicating securely the orientation of the cutting arm 50 of the second cutting device 38. The second cutting device 38 is now rotated into a position where it will change shape in such a way that the cutting arm 50 will extend to contact and be supported by the first cutting device 30, which has been inserted previously. The second cutting device 38 is advanced into a position where the atrial end 48 of the second cutting device 38 is still outside the LIPV orifice. When the correct position of the second cutting device 38 is confirmed by means of fluoroscopy and/or ultrasound, the distal end of the second cutting device 38 is released from the delivery catheter far inside the PV, whereby the distal end will expand radially to fix the position of the second cutting device 38. Next, a mid portion of the second cutting device 38 and the atrial end 48 is released, as illustrated in Fig. 20. Now, the cutting arm 50 is released, as illustrated in Fig. 21, and allowed to assume its radial extension from the tubular part 40, whereby it will penetrate the heart wall to contact the first cutting device 30.

Now, the guide wire 140 is retracted into the LA. The diagnostic catheter is inserted again and guided into the RIPV, whereby the guide wire 140 may be inserted into the RIPV. Thereafter, the diagnostic catheter is withdrawn from the patient. Then, the third cutting device 54 is inserted using a guide catheter extending to the RIPV orifice and a delivery catheter 144 in a manner similar to the insertion of the second cutting device 38. Thus, the orientation of the cutting arm 66 of the third cutting device 54 is determined in the same manner as for the second cutting device 38. Having correctly positioned the third cutting device 54, the tubular part 56, the atrial end 64 and the cutting arm 66 of the third cutting device 54 are released in a manner similar to the release of the second cutting device 38. Now, the cutting arm 66 is released and allowed to assume its radial extension from the tubular part 56, whereby it will penetrate the heart wall to contact the first cutting device 30.

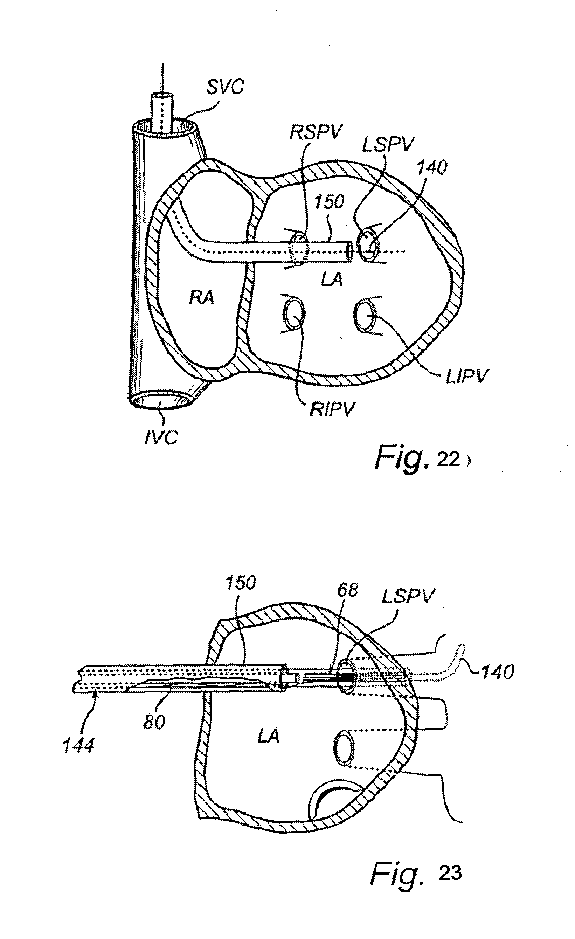

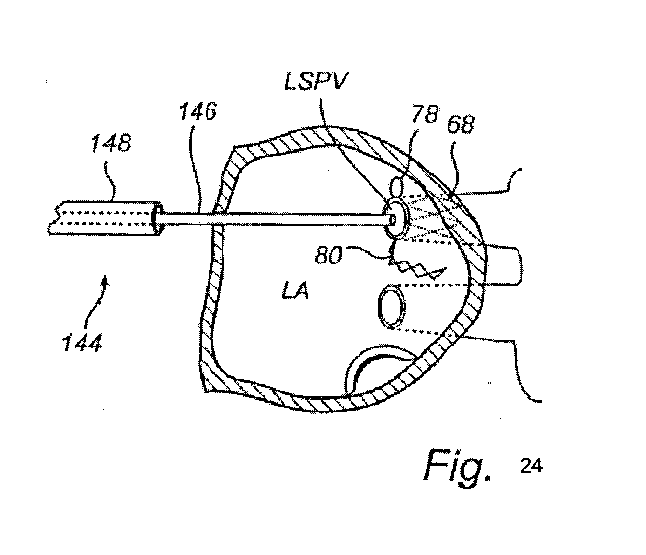

Thereafter, the guide wire 140 is again retracted into the LA and inserted into the LSPV, as illustrated in Fig. 22. Then, the fourth cutting device 68 is inserted using a guide catheter 150 extending to the LSPV orifice and a delivery catheter 144, as illustrated in Fig. 23, in a manner similar to the insertion of the second and third cutting devices 38, 54. Thus, the orientation of the cutting arm 80 of the fourth cutting device 68 is determined in the same manner as for the second and third cutting devices 38, 54. The fourth cutting device 68 may have two cutting arms, which are adapted to extend towards the second cutting device 38 and towards the LAA. Having correctly positioned the fourth cutting device 68, the tubular part 70, the atrial end 78 and the one or two cutting arms 80 of the fourth cutting device 68 are released in a manner similar to the release of the second and third cutting devices 38, 54, as further illustrated in Fig. 24. Now, the cutting arms are released and allowed to assume their radial extension from the tubular part 70, whereby they will penetrate the heart wall to contact the second cutting device 38 or extend to the orifice of the LAA, respectively.

Again, the guide wire 140 is retracted into the LA and inserted into the RSPV. Then, the fifth cutting device 82 is inserted using a guide catheter 150 extending to the RSPV orifice and a delivery catheter 144 in a manner similar to the insertion of the second, third and fourth cutting devices 38, 54, 68. Usually, the fifth cutting device 82 has no cutting arm and therefore only the axial position of the fifth cutting device 82 needs to be determined. Having correctly positioned the fifth cutting device 82, the tubular part 84, and the atrial end 92 of the fifth cutting device 82 are released in a manner similar to the release of the second, third, and fourth cutting devices 38, 54, 68.

Once again, the guide wire 140 is retracted into the LA and now inserted into the LAA. Then, the sixth cutting device 94 is inserted using a guide catheter 150 extending to the LAA orifice and a delivery catheter 144 in a manner similar to the insertion of the other cutting devices. The sixth cutting device 94 is advanced into a position where the entire sixth cutting device 94 is inside the LAA, and a proximal end of the sixth cutting device 94 is adjacent to the LAA orifice. The delivery catheter 144 has a marker on the catheter outside the patient, as well as a x-ray marker 149 visible on the fluoroscopy, indicating securely the orientation of the sixth cutting device 94 such that the elliptic shape of the sixth cutting device 94 may be oriented in correspondence to the elliptic shape of the LAA. When the correct position of the sixth cutting device 94 is confirmed by means of fluoroscopy, a distal end of the sixth cutting device 94 is released from the delivery system far inside the LAA, whereby the distal end will expand radially towards the wall of the LAA to fix the position of the sixth cutting device 94. Next, a mid portion of the sixth cutting device 94 and a proximal end are released. Now, the sixth cutting device 94 is allowed to change its shape to cut through the heart wall of the LAA.

Now, the guide wire 140 is retracted from the LA into the RA and inserted into the RAA. Then, another sixth cutting device 94 is inserted using a guide catheter 150 extending to the RAA orifice and a delivery catheter 144 in a manner similar to the insertion of the other cutting devices. The other sixth cutting device 94 is advanced into a position where the entire sixth cutting device 94 is inside the RAA, and a proximal end of the sixth cutting device 94 is adjacent to the RAA orifice. The position of the sixth cutting device 94 is determined in a manner similar to the positioning of the sixth cutting device 94 inserted into the LAA. When the correct position of the sixth cutting device 94 is confirmed, the sixth cutting device 94 inserted into the RAA is released in a manner similar to the release of the sixth cutting device 94 inserted into the LAA. Now, the sixth cutting device 94 is allowed to change its shape to cut through the heart wall of the RAA.

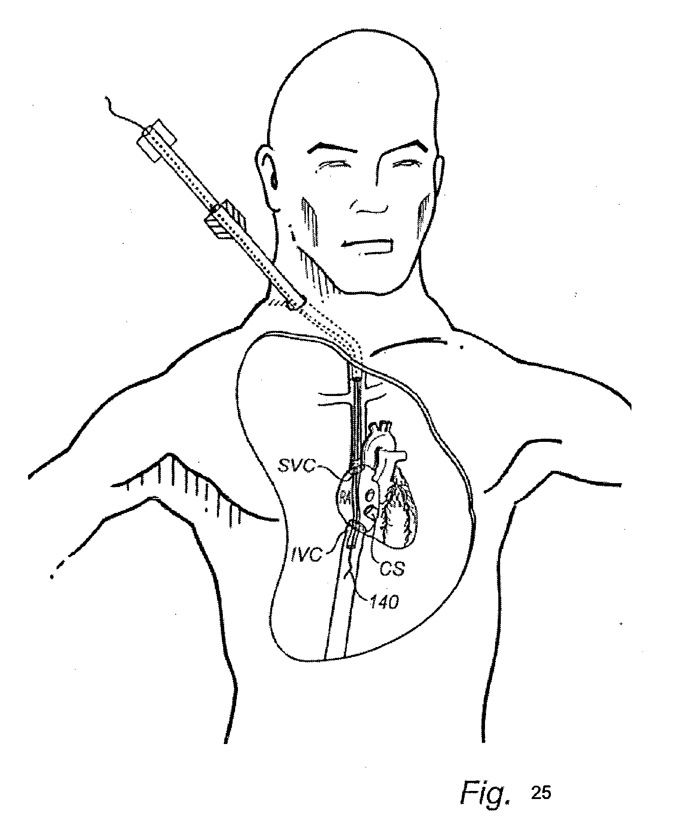

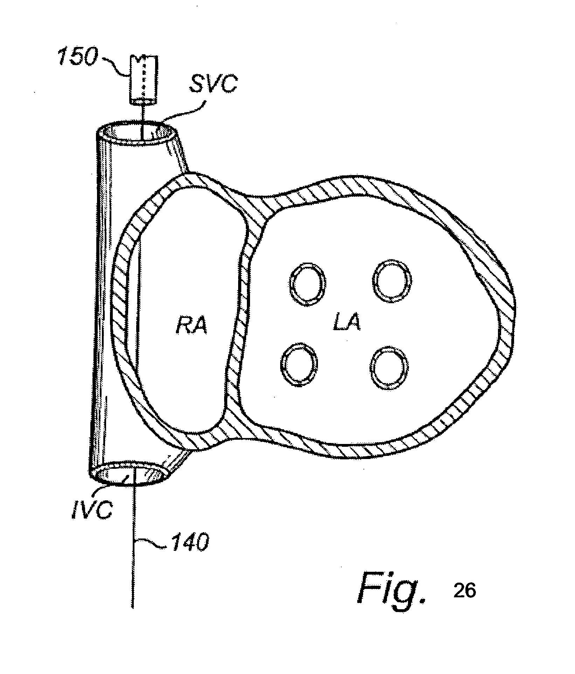

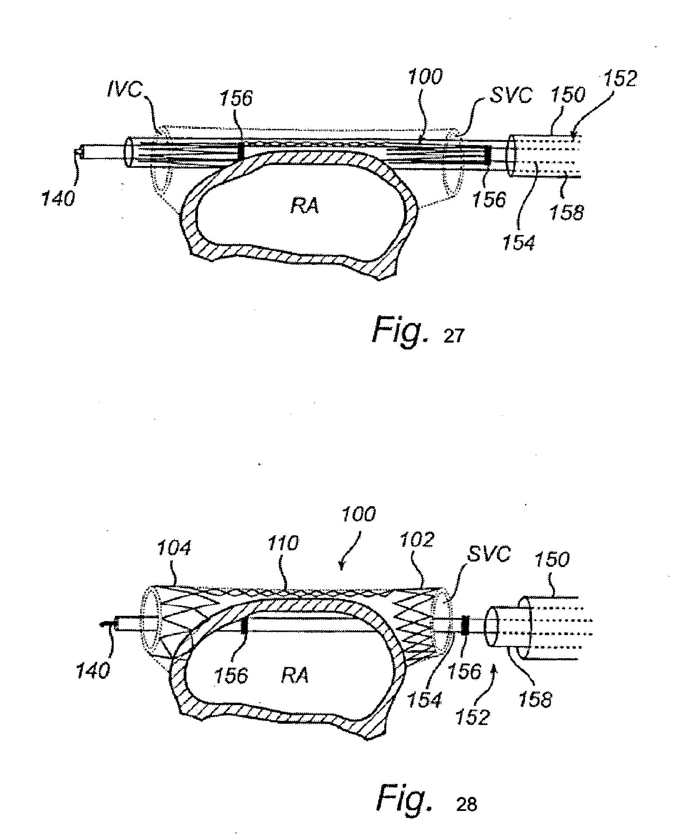

Next, the guide wire 140 is retracted from the RAA into the RA. If the access point to the vascular system was created in the upper part of the body, the guide wire 140 extends through the SVC into the RA. Then, the guide wire 140 is further inserted into the IVC, as illustrated in Fig. 25. On the other hand, if the access point to the vascular system was created in the lower part of the body, the guide wire 140 extends through the IVC into the RA. Then, the guide wire 140 is further inserted into the SVC. Thereafter, the seventh cutting device 100 is inserted using a guide catheter 150, as illustrated in Fig. 26, and a delivery catheter 144 in a manner similar to the insertion of the other cutting devices. The seventh cutting device 100 is placed in position in the IVC, SVC and the RA, as illustrated in Fig. 27. The delivery catheter 152 carries the seventh cutting device 100 on the inner part 154 of the catheter 152. The inner part 154 comprises stops 156, which prevent the seventh cutting device 100 from being axially displaced from the inner part 154 during insertion of the device. Again, the cutting device 100 is kept in a contracted, temporary state by means of a restraining part 158. The correct orientation of the seventh cutting device 100 is obtained in a manner similar to the positioning of the second, third and fourth cutting devices 38, 54, 68. The seventh cutting device 100 has now been rotated into a position where it will change shape in such a way that its cutting arm or cutting arms 122 will extend in intended directions. Thus, the seventh cutting device 100 may comprise a cutting arm 122 that extends towards the orifice of the CS and/or a branch 112 that extends from the connecting cutting arm 110 of the seventh cutting device 100 towards the lateral wall of the RA. When the correct position of the seventh cutting device 100 is confirmed by means of fluoroscopy, a distal end of the seventh cutting device 100 in the delivery catheter 152 is released from the delivery catheter 152 in the IVC or SVC, depending on where the distal end of the delivery catheter is placed. Thereafter, the connecting cutting arm 110 is released and finally a proximal end of the seventh cutting device 100 is released, as illustrated in Fig. 28.

Now, the guide wire 140 and the delivery catheter 152 is retracted outside the patient, since all parts of the treatment kit have been implanted.

On special indication, for instance when it is difficult to place the guide wire inside the PVs, an arterial access may be used instead. The insertion technique is identical, except that the access to the vascular system is achieved by puncture of an artery and that the cutting devices are delivered through the arterial system instead of through the venous system. After puncture of the artery, a catheter is advanced through the aorta and passed by the aortic valve into the left ventricle and finally into the LA. The guide wire is advanced into the desired PV and the insertion of the cutting device may then be achieved in the manner described above.

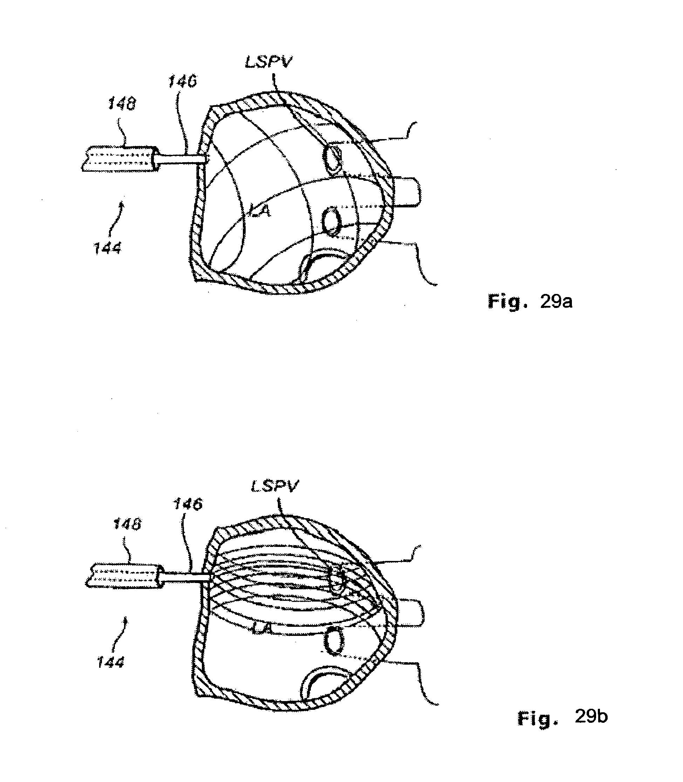

Referring now to Figs 29a and b , the release of a cutting device, according to Fig. 9, into the left atrium will be generally described. Thus, having now placed the guide wire 140, the cutting device according to Fig. 9 may be inserted to its desired position using a guide catheter extending to the LA and a delivery catheter 114, as illustrated in Fig. 19, in a similar manner as for the insertion of the first cutting device 30. The delivery catheter 144 has an inner part 146 providing the guide wire channel. The guiding catheter and the delivery catheter are advanced well into the LA so that when releasing the device into the LA the device gets contact with the wall furthest away, the guiding catheter is retracted into the RA and the restraining catheter is retracted towards the atrial septum causing the device to be released into the LA. The catheters and the guide wire are retracted to outside the patient.

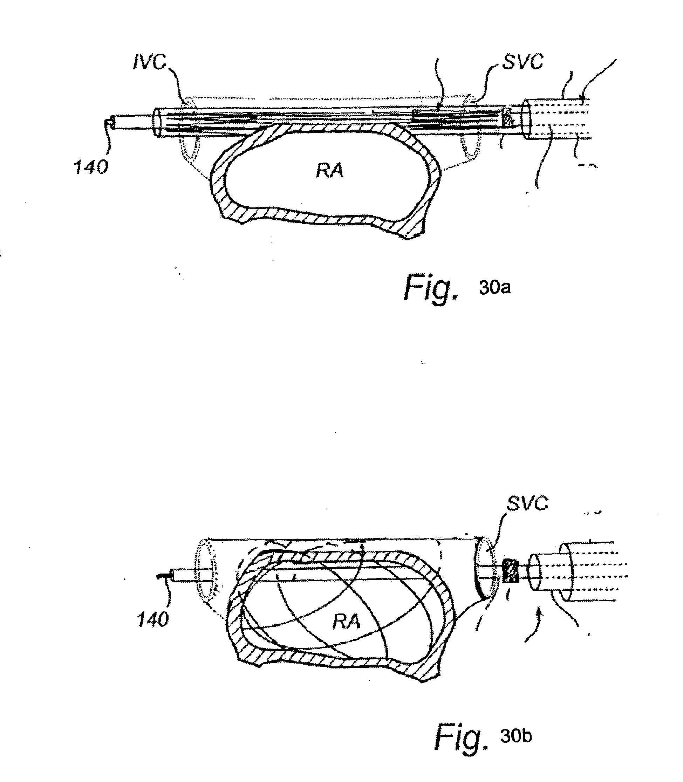

Now a release of the device in the RA is described. The guide wire is advanced into the IVC if the approach is from the neck and into the SVC if the approach is from the groin, according to Fig 30a and b. The delivery catheter is advanced to the most distant point where the atrial device is to be deploid, the restraining catheter is retracted towards the SVC or IVC respectively, causing the device to be released into the RA, according to Fig. 30b. The catheters and the guide wire are retracted to outside the patient.

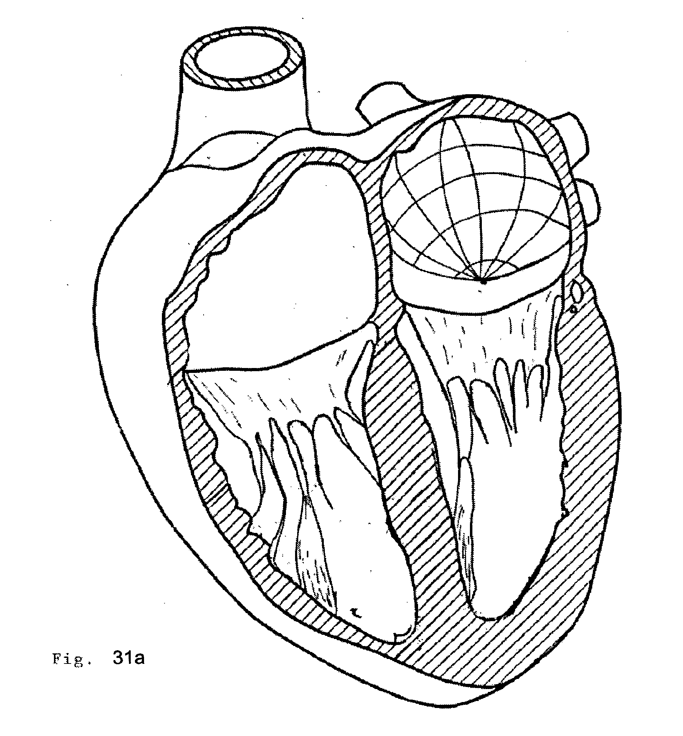

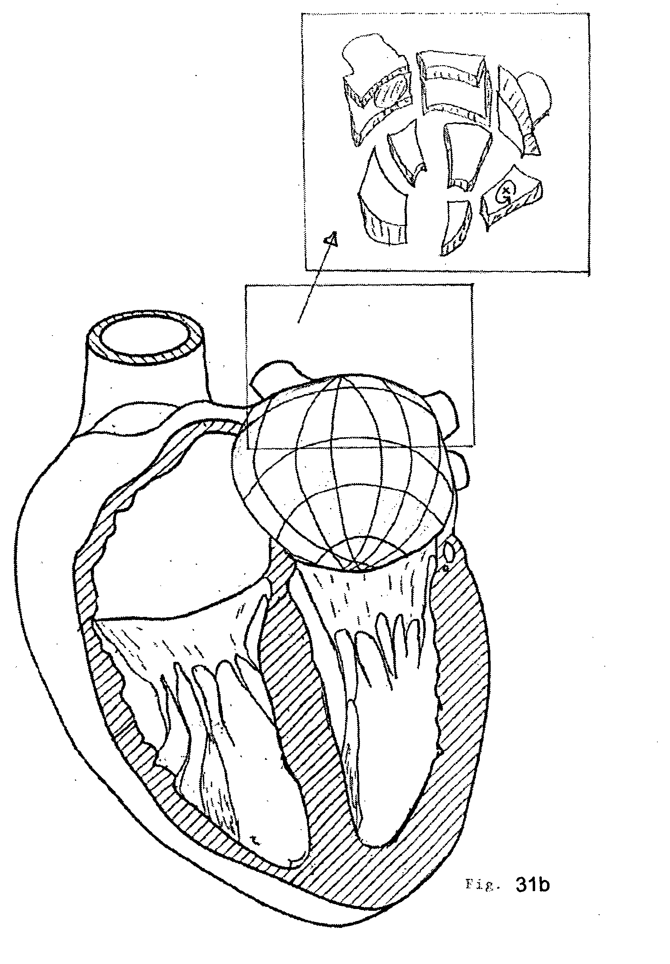

Fig. 31a shows the cutting device according to Fig 9a positioned in the RA, and Fig 31b shows the same cutting device in the permanent, expanded shape, i.e. when the wall of the RA has been cut.

The cutting devices according to the present invention have now been released such that they may change their shapes to obtain their permanent shapes. During the change of shape, each cutting device will penetrate heart tissue in the path of the change of shape. Thus, the cutting devices will now create the cutting pattern intended for forming blocks against propagation of undesired electrical signals in the heart, until the connection material is absorbed or broken, by for example stress, to thereby interrupt the expansion. After the cutting devices have made their change of shape, the needed effect of the cutting devices on the heart tissue is completed. Thus, if the cutting devices are made of resorbable shape memory polymers, the cutting devices will be resorbed a time after termination of the cutting procedure. This time for resorption can be set by determination of the different ingredients of polymers and also by means of external altering, for instance by means of x-ray radiation, ultrasound, electron beams, or light of a defined wavelength, setting the time of the polymers to be resorbed. However, the cutting devices may also be left in the body after the change of shape, or only some of the cutting devices may be resorbed.

Moreover, other design parameters of tissue cutting devices may be chosen according to patient specific anatomy. Such design parameters are for instance wire thickness distribution, connection points, fastening elements such as hooks, bistable sections or characteristics, material choice, implementation of drug delivery sections, timing design of cutting action, etc. as described in co-pending patent applications concurrently filed by same applicant as present application, which hereby are incorporated by reference herein in their entirety.

Hereinafter, some potential uses of the device of the present invention are described:
A method for treatment of disorders in the heart rhythm regulation system, said method comprising:
   inserting a tissue cutting device through the vascular system to a desired position in a body vessel, and providing a change of shape of the tissue cutting device at said desired position to penetrate heart tissue adjacent said body vessel, until a connecting material, connecting parts of the cutting device, is absorbed or broken, whereby the cutting action is interrupted.

The method according to above, wherein said tissue cutting device is inserted into a desired position in the coronary sinus, in any of the pulmonary veins, in the superior vena cava, in the inferior vena cava, or in the left or right atrial appendage.

The method according to above, further comprising inserting another tissue cutting device to another of the desired positions.

The method according to above, further comprising inserting a tissue cutting device into each of the desired positions.

The method according to above, further comprising restraining the tissue cutting device in an insertion shape during the inserting of the tissue cutting device.

The method according to above, wherein the restraining comprises keeping the tissue cutting device inside a tube.

The method according to above, wherein the restraining comprises cooling the tissue cutting device.

The method according to above, further comprising releasing a restrain on the tissue cutting device when it has been inserted into the desired position for allowing said change of the shape of the tissue cutting device.

It should be emphasized that the preferred embodiments described herein is in no way limiting and that many alternative embodiments are possible within the scope of protection defined by the appended claims.

## Claims

1. A tissue cutting device (26, 30, 38, 54, 68, 82,94, 100) configured for reducing undesired signal transmission in a heart tissue (2) by isolating ectopic sites thereof by cutting said tissue,
wherein the device is structured and arranged to be inserted in a temporary delivery shape through the vascular system into a body vessel adjacent to the heart and/or into the heart and to be subsequently subjected to a change of shape, from said temporary delivery shape via an expanded delivered shape to a further expanded shape, in order to create cutting action configured for cutting said heart tissue and/or said body vessel, wherein said tissue cutting device is adapted to penetrate through a wall of said body vessel or said heart tissue and destroy the tissue in order to prevent it from transmitting electrical signals; and **characterized in that** said tissue cutting device comprises
means for regulating said cutting action, which is adapted to stop said cutting action upon de-activation of said means for regulating said cutting action,
wherein said means for regulating said cutting action comprises at least one connection element that is arranged such that a connection formed by said connection element between a first and second part (A, B) of said tissue cutting device is configured to break when said connection element is subjected to a specific external influence, wherein the breaking of that connection element is configured to provide said de-activation of at least a part of said cutting device for said cutting action.

2. The tissue cutting device according to claim 1,
wherein the tissue cutting device and/or said connection element is at least partly made of a biodegradable, bioresorbable, or bioabsorbable material.

3. The tissue cutting device according to any of the preceding claims, wherein the device has an initial elongate shape and wherein the device is structured and arranged to change shape to expand its dimensions in a radially outward oriented direction thereof, to thereby provide said cutting action for said heart tissue and/or body vessel.

4. The tissue cutting device according to claim 1, wherein said cutting device has a globular shape, and wherein said cutting device comprises intersecting points of strands of at least one wire, wherein at least a part of said intersecting points are connected by means of said at least one connection element.

5. The tissue cutting device according to any of claims 1 or 2, wherein said device has a spiral shape comprising at least two spiral fragments (C, D), connected with said connection element.

6. The tissue cutting device as claimed in any one of the preceding claims, wherein the device comprises a shape memory material configured for achieving said change of shape from a temporary delivery shape to an expanded delivered shape, preferably in an atrium of said heart.

7. The tissue cutting device as claimed in any one of the preceding claims, wherein said device is constituted of wires with a first diameter (d) and said connection point is a part of said wire with a second diameter (D), wherein said first diameter is greater than said second diameter.

8. The tissue cutting device according to claim 1, wherein said tissue cutting device is configured for reducing undesired signal transmission in a heart tissue by isolating ectopic sites thereof by cutting said tissue,
wherein the device is structured and arranged to be inserted in a temporary delivery shape through the vascular system into a body vessel adjacent to the heart, and to be subsequently subjected to a change of shape, from said temporary delivery shape via an expanded delivered shape to a further expanded shape, in order to create cutting action configured for cutting said heart tissue and/or said body vessel, wherein the device comprises a plurality of segments connected to each other in longitudinal direction of said device, wherein a first segment of said plurality of segments has a dimension in a direction perpendicular to said longitudinal direction of said device larger than that dimension of a second segment thereof, at least in said expanded delivered shape, and wherein the device comprises at least one connection element, arranged such that the connection formed from said connection element between a first and second part of said cutting device is configured to break when said connection element is subjected to a specific external influence.

9. The tissue cutting device according to claim 8, wherein said device further comprises at least one cutting arm (50, 66, 80, 110, 122) being structured and arranged to initially extend substantially perpendicular to said longitudinal direction from the tissue cutting device in order to be inserted into a heart atrium wall and said cutting arm being structured and arranged to change shape to extend radially from the tissue cutting device, wherein said at least one connection point is located within said arm(s) and/or between said arm(s) and said cutting device.

10. The tissue cutting device as claimed in any one of the preceding claims, wherein said device has a net-like shape formed of closed loops.

11. A tissue cutting device according to claim 1,
wherein the device is structured and arranged to be inserted into a body vessel and to subsequently change shape, wherein the device is structured and arranged to change shape to extend at least partly outside the perimeter or orifice of an outer wall of said vessel in said further expanded shape, and wherein the device comprises at least one connection element, arranged such that the connection formed from said connection element between a first (A) and second part (B) of said cutting device is configured to break when said connection element is subjected to a specific external influence.

12. The tissue cutting device according to any of claims 1-11, wherein said specific external influence comprises:
stress, temperature, moisture, biodegration, or absorption.

13. A kit of shape-changing tissue cutting devices (26, 30, 38, 54, 68, 82,94, 100) according to claims 1 to 12 for treatment of disorders in the heart rhythm regulation system, said kit comprising:
a plurality of said shape-changing tissue cutting devices, which each has a first delivery and a second delivered state, wherein each of the tissue cutting devices in the first state has such dimensions as to be insertable to a desired position within the vascular system, and wherein each of the devices is capable of changing shape to substantially the second state when located at said desired position, which strives to a diameter that is larger than the diameter of the vessel at the desired position, whereby each of the devices will become embedded into the tissue surrounding the vessel at the desired position and destroy the tissue in order to prevent it from transmitting electrical signals,
wherein at least one of the plurality of shape-changing tissue cutting devices is adapted to be inserted to a desired position at the orifice of a pulmonary vein in the heart, and at least one of the plurality of shape-changing tissue cutting devices is adapted to be inserted to a desired position in the coronary sinus, and wherein the at least a first tissue cutting device of the plurality of shape-changing tissue cutting devices comprises at least one connection element, arranged such that the connection formed from said connection element between a first and second part (A, B) of said first tissue cutting device is configured to break when said connection element is subjected to a specific external influence.

14. The kit as claimed in any one claim 13, wherein at least one of the shape-changing devices is adapted to be inserted into the inferior vena cava, or wherein at least one of the shape-changing devices is adapted to be inserted into the superior vena cava.

## Patentansprüche

1. Gewebeschneidegerät (26, 30, 38, 54, 68, 82, 94, 100), welches so konfiguriert ist, dass es der Reduktion von unerwünschter Signalübertragung in einem Herzgewebe (2) dient, indem dessen ektopische Bereiche mittels der Durchtrennung dieses Gewebes isoliert werden,
wobei das Gerät so strukturiert und angeordnet ist, dass es in einer vorübergehenden Beförderungsform durch das Gefäßsystem in ein dem Herzen benachbartes Körpergefäß und/oder in das Herz eingeführt werden kann, und welches nachfolgend einer Formveränderung unterworfen wird, die von der zeitweiligen Beförderungsform über eine erweiterte beförderte Form zu einer weiter erweiterten Form führt, so dass ein Schneidevorgang bewirkt wird, der zum Schneiden des Herzgewebes und/oder des Körpergefäßes konfiguriert ist, wobei das Gefäßschneidegerät so angepasst ist, dass es die Wand des Körpergefäßes oder des Herzgefäßes durchdringt und das Gewebe zerstört, um zu vermeiden, dass dieses elektrische Signale überträgt; und wobei das Gewebeschneidegerät **dadurch** charakterisiert ist, dass es Folgendes umfasst:
Mittel zur Regulierung des Schneidevorgangs, welches so angepasst ist, dass es den Schneidevorgang abbricht, sobald das Mittel zur Regulierung der Schneidevorgangs deaktiviert wird,
wobei das Mittel zur Regulierung des Schneidevorgangs mindestens ein Verbindungselement umfasst, welches so angeordnet ist, dass eine Verbindung, welche durch das Verbindungselement zwischen einem ersten und einem zweiten Teil (A, B) des Gewebeschneidegeräts hergestellt wird, so konfiguriert ist, dass sie unterbrochen wird, wenn das Verbindungselement einem spezifischen äußeren Einfluss ausgesetzt wird, wobei das Unterbrechen dieses Verbindungselements so konfiguriert ist, dass es die Deaktivierung von mindestens einem Teil des dem Schneidevorgang dienenden Gewebeschneidegeräts bewirkt.

2. Gewebeschneidegerät gemäß Anspruch 1, wobei das Gewebeschneidegerät und/oder das Verbindungselement zumindest teilweise aus bioabbaubarem, bioresorbierbarem oder bioabsorbierbarem Material besteht.

3. Gewebeschneidegerät gemäß einem beliebigen der vorangegangenen Ansprüche, wobei die Vorrichtung eine anfängliche verlängerte Form aufweist, und wobei das Gerät so strukturiert und angeordnet ist, dass es seine Form verändert, so dass es seine Ausmaße in eine in Bezug auf sich selbst radial auswärts verlaufende Richtung ausdehnt, so dass **dadurch** der Schneidevorgang für das besagte Herzgewebe und/oder Körpergefäß erfolgt.

4. Gewebeschneidegerät gemäß Anspruch 1, wobei das Schneidegerät eine kugelförmige Form aufweist, und wobei die Schneidevorrichtung sich überschneidende Punkte von Strängen von zumindest einem Draht umfasst, wobei zumindest ein Teil der sich überschneidenden Punkte mittels mindestens eines Verbindungselementes verbunden wird.

5. Gewebeschneidegerät gemäß Anspruch 1 oder 2, wobei das Gerät eine spiralartige Form aufweist, die aus mindestens zwei Spiralfragmenten (C, D) besteht, welche mit dem Verbindungselement verbunden sind.

6. Gewebeschneidegerät gemäß einem beliebigen der vorangegangenen Ansprüche, wobei das Gerät ein Formgedächtnismaterial umfasst, welches so konfiguriert ist, dass es die Formveränderung von einer zeitweiligen Beförderungsform zu einer erweiterten beförderten Form erreicht, und zwar vorzugsweise in einem Vorhof des Herzens.

7. Gewebeschneidegerät gemäß einem beliebigen der vorangegangenen Ansprüche, wobei das Gerät aus Drähten mit einem ersten Durchmesser (d) besteht und der Verbindungspunkt einen Teil des Drahtes mit einem zweiten Durchmesser (D) bildet, wobei der erste Durchmesser größer als der zweite Durchmesser ist.

8. Gewebeschneidegerät gemäß Anspruch 1, wobei das Gewebeschneidegerät so konfiguriert ist, dass es der Reduktion von unerwünschter Signalübertragung in einem Herzgewebe dient, indem dessen ektopische Bereiche **dadurch** isoliert werden, dass das Gewebe durchschnitten wird,
wobei das Gerät so strukturiert und angeordnet ist, dass es in einer vorübergehenden Beförderungsform durch das Gefäßsystem in ein neben dem Herzen liegendes Körpergefäß eingeführt werden kann, und welches nachfolgend einer Formveränderung von der zeitweiligen Beförderungsform über eine erweiterte beförderte Form zu einer weiter erweiterten Form unterworfen wird, um eine Schneidevorgang zu bewirken, der zum Schneiden des Herzgewebes und/oder des Körpergefäßes konfiguriert ist, wobei das Gerät eine Vielzahl von Segmenten umfasst, welche in Längsrichtung des Gerätes miteinander verbunden sind, wobei ein erstes Segment aus der Vielzahl von Segmenten eine Ausdehnung in einer Richtung aufweist, welche senkrecht zu der Längsrichtung des Gerätes verläuft und - zumindest in der erweiterten beförderten Form - größer ist als die Dimension eines zweiten von dessen Segmenten, und wobei das Gerät mindestens ein Verbindungselement umfasst, welches so angeordnet ist, dass die Verbindung, die durch das Verbindungselement zwischen einem ersten und einem zweiten Teil des Schneidegeräts hergestellt wird, so konfiguriert ist, dass sie unterbrochen wird, wenn das Verbindungselement einem spezifischen äußeren Einfluss ausgesetzt äußeren Einfluss ausgesetzt wird.

9. Gewebeschneidegerät gemäß Anspruch 8, wobei das Gerät ferner mindestens einen Schneidarm (50, 66, 80, 110, 122) umfasst, welcher so strukturiert und angeordnet ist, dass er sich anfangs im Wesentlichen senkrecht zur Längsrichtung vom Gewebeschneidegerät weg erstreckt, um in einen Herzvorhofswand eingeführt zu werden, wobei der Schneidarm so strukturiert und angeordnet ist, dass er seine Form verändert, so dass er sich radial von dem Gewebeschneidegerät weg erstreckt, wobei der mindestens eine Verbindungspunkt sich innerhalb des Arms (bzw. der Arme) und/oder zwischen dem Arm (bzw. den Armen) und der Schneidevorrichtung befindet.

10. Gewebeschneidegerät gemäß einem beliebigen der vorangegangenen Ansprüche, wobei das Gerät eine netzartige Form aufweist, welche aus geschlossenen Schlaufen gebildet wird.

11. Gewebeschneidegerät gemäß Anspruch 1, wobei das Gerät so strukturiert und angeordnet ist, dass es in ein Körpergefäß eingeführt werden kann und nachfolgend die Form verändert, wobei das Gerät so strukturiert und angeordnet ist, dass es die Form verändert, so dass es sich in seiner weiter erweiterten Form mindestens teilweise bis außerhalb des Umfangs oder der Öffnung einer äußeren Wand des Gefäßes erstrecken kann, und wobei die Vorrichtung mindestens ein Verbindungselement umfasst, welches so angeordnet ist, dass die Verbindung, welche durch das Verbindungselement zwischen einem ersten (A) und einem zweiten Teil (B) des Schneidegeräts hergestellt wird, so konfiguriert ist, dass sie unterbrochen wird, wenn das Verbindungselement einem spezifischen äußeren Einfluss ausgesetzt wird.

12. Gewebeschneidegerät gemäß einem beliebigen der Ansprüche 1-11, wobei der spezifische äußere Einfluss umfasst:
Belastung, Temperatur, Feuchtigkeit, Bioabbau oder Absorption.

13. Ein Gerätesatz von formverändernden Gewebeschneidegeräten (26, 30, 38, 54, 68, 82, 94, 100) gemäß Ansprüchen 1 bis 12 zur Behandlung von Störungen des Herzrhythmusreguliersystems, wobei der Gerätesatz umfasst:
Vielzahl von formverändernden Gewebeschneidegeräten, von denen jedes einen ersten Beförderungs- und einen zweiten beförderten Zustand hat, wobei jedes der Gewebeschneidegeräte im ersten Zustand solche Dimensionen hat, dass es in eine gewünschte Position innerhalb des vaskulären Systems eingeführt werden kann, und wobei jedes der Geräte seine Form im Wesentlichen zu dem zweiten Zustand hin verändern kann, wenn es sich in der gewünschten Position befindet, wobei ein Durchmesser angestrebt wird, welcher größer ist als der Durchmesser des Gefäßes an der gewünschten Position, wobei jedes der Geräte in das Gewebe, welches das Gefäß an der gewünschten Position umgibt, eingebettet wird, und das Gewebe zerstört, um zu verhindern, dass es elektrische Signale überträgt,
wobei mindestens eines der Vielzahl von formverändernden Gewebeschneidegeräten so angepasst ist, dass es an eine gewünschte Position an der Öffnung einer Pulmonalvene im Herzen eingeführt werden kann, und mindestens eines der formverändernden Gewebeschneidegeräte so angepasst ist, dass es an eine gewünschte Position in dem Koronarsinus eingeführt werden kann, und wobei mindestens ein erstes Gewebeschneidegerät aus der Vielzahl der formverändernden Gewebeschneidegeräte mindestens ein Verbindungselement umfasst, welches so angeordnet ist, dass die Verbindung, welche durch das Verbindungselement zwischen einem ersten und zweiten Teil (A, B) des ersten Gewebeschneidegerätes hergestellt wird, so konfiguriert ist, dass sie unterbrochen wird, wenn das Verbindungselement einem spezifischen äußeren Einfluss ausgesetzt wird.

14. Gerätesatz gemäß Anspruch 13, wobei mindestens eines der formverändernden Geräte so angepasst ist, dass es in die Vena cava inferior eingeführt werden kann, oder wobei mindestens eines der formverändernden Geräte so angepasst ist, dass es in die Vena cava superior eingeführt werden kann.

## Revendications

1. Dispositif de découpe de tissu (26, 30, 38, 54, 68, 82 94, 100) configuré afin de réduire une transmission de signal non souhaitée dans un tissu cardiaque (2) en isolant des sites ectopiques de celui-ci par découpe dudit tissu,
où le dispositif est structuré et agencé de manière à être inséré sous une forme de délivrance temporaire à travers le système vasculaire jusque dans un vaisseau sanguin adjacent au coeur et/ou jusque dans le coeur et de manière à être par la suite soumis à un changement de forme, à partir de ladite forme de délivrance temporaire en passant par une forme délivrée déployée jusqu'à une forme davantage déployée, de manière à créer une action de découpe configurée afin de découper ledit tissu cardiaque et/ou ledit vaisseau sanguin, où le dispositif de découpe de tissu est adapté pour pénétrer à travers une paroi dudit vaisseau sanguin ou dudit tissu cardiaque et pour détruire le tissu de manière à l'empêcher de transmettre des signaux électriques, et **caractérisé en ce que** ledit dispositif de découpe de tissu comprend
un moyen destiné à réguler ladite action de découpe, qui est adapté pour stopper ladite action de découpe lors d'une désactivation dudit moyen destiné à réguler ladite action de découpe,
où ledit moyen destiné à réguler ladite action de découpe comprend au moins un élément de connexion qui est agencé de telle sorte qu'une connexion formée par ledit élément de connexion entre une première et une deuxième parties (A, B) dudit dispositif de découpe de tissu est configurée pour se rompre lorsque ledit élément de connexion est soumis à une influence externe spécifique, où la rupture de cet élément de connexion est configurée pour fournir ladite désactivation d'au moins une partie dudit dispositif de découpe pendant ladite action de découpe.

2. Dispositif de découpe de tissu selon la revendication 1, où le dispositif de découpe de tissu et/ou ledit élément de connexion sont au moins partiellement constitués d'un matériau biodégradable, biorésorbable, ou bio-absorbable.

3. Dispositif de découpe de tissu selon l'une quelconque des revendications précédentes, où le dispositif présente une forme allongée initiale et où le dispositif est structuré et agencé pour changer de forme afin de déployer ses dimensions suivant une direction orientée radialement vers l'extérieur de celui-ci, afin de fournir de cette manière ladite action de découpe pour ledit tissu cardiaque et/ou ledit vaisseau sanguin.

4. Dispositif de découpe de tissu selon la revendication 1, où ledit dispositif de découpe présente une forme globulaire, et où ledit dispositif de découpe comprend des points d'intersection de brins d'au moins un fil, où au moins une partie desdits points d'intersection sont connectés au moyen dudit au moins un élément de connexion.

5. Dispositif de découpe de tissu selon l'une quelconque des revendications 1 ou 2, où ledit dispositif présente une forme en spiral comprenant au moins deux fragments en spiral (C, D), connectés audit élément de connexion.

6. Dispositif de découpe de tissu selon l'une quelconque des revendications précédentes, où le dispositif comprend un matériau à mémoire de forme configuré pour obtenir ledit changement de forme à partir d'une forme de délivrance temporaire jusqu'à une forme délivrée déployée, de préférence dans une oreillette dudit coeur.

7. Dispositif de découpe de tissu selon l'une quelconque des revendications précédentes, où ledit dispositif est constitué de fils présentant un premier diamètre (d) et ledit point de connexion est une partie dudit fil présentant un second diamètre (D), où ledit premier diamètre est supérieur audit deuxième diamètre.

8. Dispositif de découpe de tissu selon la revendication 1, où ledit dispositif de découpe de tissu est configuré afin de réduire une transmission de signal non souhaitée dans un tissu cardiaque en isolant des sites ectopiques de celui-ci par découpe dudit tissu,
où le dispositif est structuré et agencé de manière à être inséré suivant une forme de délivrance temporaire au travers du système vasculaire jusque dans un vaisseau sanguin adjacent au coeur, et de manière à être par la suite soumis à un changement de forme, à partir de ladite forme de délivrance temporaire en passant par une forme délivrée déployée jusqu'à une forme davantage déployée, de manière à créer une action de découpe configurée afin de découper ledit tissu cardiaque et/ou ledit vaisseau sanguin, où le dispositif comprend une pluralité de segments connectés les uns aux autres suivant une direction longitudinale dudit dispositif, où un premier segment de ladite pluralité de segments présente une dimension suivant une direction perpendiculaire à ladite direction longitudinale dudit dispositif supérieure à cette dimension d'un deuxième segment de celui-ci, au moins dans ladite forme délivrée déployée, et où le dispositif comprend au moins un élément de connexion, agencé de telle sorte que la connexion formée à partir dudit élément de connexion entre une première et une deuxième parties dudit dispositif de découpe est configurée pour se rompre lorsque ledit élément de connexion est soumis à une influence externe spécifique.

9. Dispositif de découpe de tissu selon la revendication 8, où ledit dispositif comprend en outre au moins un bras de découpe (50, 66, 80, 110, 122) qui est structuré et agencé pour s'étendre initialement pratiquement perpendiculairement à ladite direction longitudinale à partir du dispositif de découpe de tissu de manière à être inséré dans une paroi d'oreillette cardiaque et ledit bras de découpe étant structuré et agencé afin de changer de forme pour s'étendre radialement à partir du dispositif de découpe de tissu, où ledit au moins un point de connexion est situé au sein dudit ou desdits bras et/ou entre ledit ou lesdits bras et ledit dispositif de découpe.

10. Dispositif de découpe de tissu selon l'une quelconque des revendications précédentes, où ledit dispositif présente une forme de type réseau formée de boucles fermées.

11. Dispositif de découpe de tissu selon la revendication 1, où le dispositif est structuré et agencé de manière à être inséré jusque dans un vaisseau sanguin et à changer par la suite de forme, où le dispositif est structuré et agencé pour changer de forme de manière à s'étendre au moins partiellement vers l'extérieur du périmètre ou de l'orifice d'une paroi extérieure dudit vaisseau suivant ladite forme davantage déployée, et où le dispositif comprend au moins un élément de connexion, agencé de telle sorte que la connexion formée à partir dudit élément de connexion entre une première (A) et une deuxième (B) parties dudit dispositif de découpe est configurée pour se rompre lorsque ledit élément de connexion est soumis à une influence externe spécifique.

12. Dispositif de découpe de tissu selon l'une quelconque des revendications 1 à 11, où ladite influence externe spécifique comprend :
une contrainte, une température, une humidité, une biodégradation, ou une absorption.

13. Nécessaire de dispositifs de découpe de tissu changeant de forme (26, 30, 38, 54, 68, 82, 94, 100) selon les revendications 1 à 12, destiné à un traitement de troubles du système de régulation du rythme cardiaque, ledit nécessaire comprenant :
une pluralité desdits dispositifs de découpe de tissu changeant de forme, dont chacun présente un premier état de délivrance et un deuxième état délivré, où chacun des dispositifs de découpe de tissu dans le premier état présente des dimensions telles qu'ils peuvent être insérés à une position souhaitée au sein du système vasculaire et où chacun des dispositifs est en mesure de changer de forme jusqu'à atteindre pratiquement le deuxième état lorsqu'ils sont situés au niveau de ladite position souhaitée, lesquels luttent pour obtenir un diamètre qui soit supérieur au diamètre du vaisseau au niveau de la position souhaitée, grâce à quoi chacun des dispositifs évoluera pour s'inclure dans le tissu entourant le vaisseau au niveau de la position souhaitée et détruira le tissu de manière à l'empêcher de transmettre des signaux électriques,
où au moins un dispositif parmi la pluralité de dispositifs de découpe de tissu changeant de forme est adapté de manière à être inséré à une position souhaitée au niveau de l'orifice d'une veine pulmonaire dans le coeur, et au moins un dispositif parmi la pluralité de dispositifs de découpe de tissu changeant de forme est adapté de manière à être inséré à une position souhaitée dans le sinus coronaire, et où le au moins un premier dispositif de découpe de tissu parmi la pluralité de dispositifs de découpe de tissu changeant de forme comprend au moins un élément de connexion, agencé de telle sorte que la connexion formée à partir dudit élément de connexion entre une première et une deuxième parties (A, B) dudit premier dispositif de découpe de tissu est configurée pour se rompre lorsque ledit élément de connexion est soumis à une influence externe spécifique.

14. Nécessaire selon la revendication 13, où au moins un dispositif parmi les dispositifs changeant de forme est adapté de manière à être inséré jusque dans la veine cave inférieure, ou où au moins un dispositif parmi les dispositifs changeant de forme est adapté de manière à être inséré dans la veine cave supérieure.
